# FASCICULE DE BREVET EUROPEEN

(11) **EP 1 151 121 B1**
(45) Date de publication et mention de la délivrance du brevet: **25.01.2006**
(21) Numéro de dépôt: 00903750.8
(22) Date de dépôt: 08.02.2000
(51) Int. Cl.: C12N 15/86, C12N 15/34, A61K 48/00

(54) **VECTEURS ET VACCINS VIRAUX A BASE D'ADENOVIRUS PORCINS RECOMBINES**
VEKTOREN UND VIRALE IMPFSTOFFE BASIEREND AUF REKOMBINANTEM SCHWEINE-ADENOVIRUS
RECOMBINED PORCINE ADENOVIRUS BASED VIRAL VACCINES AND VECTORS

(30) Priorité: 11.02.1999 FR 9901813
(43) Date de publication de la demande: 07.11.2001
(73) Titulaire: MERIAL, 69002 Lyon (FR); Ecole Nationale Vétérinaire de Maisons ALFORT, 94700 Maisons Alfort (FR)
(72) Inventeur: ELOIT, Marc, F-94100 St Maur des Fossés (FR); KLONJKOWSKI, Bernard, Georges, F-75012 Paris (FR)
(74) Mandataire: Nargolwalla, Cyra
(86) Numéro de dépôt international: PCT/FR2000/000294
(87) Numéro de publication internationale: WO 2000/047756

(56) Documents cités:
- WO-A-97/20036
- WO-A-99/08706
- WO-A-99/53047
- MITTAL ET AL.: "Development of a bovine adenovirus type 3-based expression vector" JOURNAL OF GENERAL VIROLOGY, vol. 76, 1995, pages 93-102, XP002087288 ISSN: 0022-1317
- TUBOLY T. ET AL.: "Restriction endonuclease analysis and physical mapping of the genome of porcine adenovirus type 5." VIRUS RESEARCH, vol. 37, no. 1, 1995, pages 49-54, XP000866400
- REDDY P. S. ET AL.: "Restriction endonuclease analysis and molecular cloning of porcine adenovirus type-3" INTERVIROLOGY, vol. 36, no. 3, 1993, pages 161-168, XP002120928 ISSN: 0300-5526 cité dans la demande
- REDDY P. S. ET AL.: "Sequence analysis of putative pVIII, E3 and fibre regions of porcine adenovirus type 3" VIRUS RESEARCH, vol. 36, no. 1, avril 1995 (1995-04), pages 97-106, XP002120929 ISSN: 0168-1702 cité dans la demande
- REDDY P. S. ET AL.: "Porcine adenoviruses types 1, 2 and 3 have short and simple early E-3 regions" VIRUS RESEARCH, vol. 43, no. 2, 1996, pages 99-109, XP002120926 ISSN: 0168-1702 cité dans la demande
- KLEIBOEKER S. B.: "Sequence analysis of putative E3, pVIII, and fiber genomic regions of a porcine adenovirus." VIRUS RESEARCH, vol. 31, no. 1, 1994, pages 17-25, XP002127600 cité dans la demande
- TORRES J. M. ET AL.: "TROPISM OF HUMAN ADENOVIRUS TYPE 5-BASED VECTORS IN SWINE AND THEIRABILITY TO PROTECT AGAINST TRANSMISSIBLE GASTROENTERITIS CORONAVIRUS" JOURNAL OF VIROLOGY, vol. 70, no. 6, juin 1996 (1996-06), pages 3770-3780, XP000616232 ISSN: 0022-538X
- REDDY P. S. ET AL.: "Development of porcine adenovirus-3 as an expression vector" JOURNAL OF GENERAL VIROLOGY, vol. 80, no. 3, mars 1999 (1999-03), pages 563-570-570, XP002120921 ISSN: 0022-1317

## Description

La présente invention a trait à des adénovirus porcins de sérotypes 3 et 5 recombinés par insertion de séquences nucléotidiques hétérologues, ces adénovirus étant aptes à se répliquer *in vivo* de manière autonome et exprimer ces séquences hétérologues. L'invention a aussi trait aux vaccins porcins obtenus, aux méthodes d'immunisation des porcs les utilisant, à des vecteurs adénovirus délétés et réplicatifs, aux méthodes d'obtention de ces adénovirus, vaccins et vecteurs.

Les documents cités dans la description, ainsi que les documents cités dans ces documents, sont incorporés ici par référence.

Les adénovirus sont des virus à molécule d'ADN double brin linéaire, avec des séquences inversées terminales répétées à chaque extrémité. Les adénovirus font partie de la famille des Adenoviridae, responsables de maladies touchant un grand nombre d'espèces, telles que les espèces simiennes, bovine, ovine, porcine, équine, murine, canine et aviaires. Les infections à adénovirus peuvent se caractériser, selon les espèces et selon les types d'adénovirus, par des signes d'encéphalites, de pneumonies, de lésions rénales, de diarrhées et d'hépatites.

Le génome des adénovirus est variable d'une espèce à l'autre [(T. Adrian et al., Arch. Virol. 1986, 91, 277-290), (J. Hamelin et al., J. Clin. Microbiol. 1988, 26,31-33), (R. Assaf et al., Can. J. Comp. Med. 1983, 47, 460-463), (T. Kurokawa et al., J. Virol. 1978, 28, 212-218), (S.-L. Hu et al., J. Virol. 1984, 51, 880-883), (L. Zsak et al., Intervirology 1984, 22, 110-114)]. Les adénovirus des animaux, contrairement aux adénovirus humains, ont un spectre d'hôte très limité et ils n'infectent souvent que les animaux appartenant à leur espèce d'origine.

Le premier adénovirus porcin (en anglais "Porcine AdenoVirus" ou PAV) a été isolé en 1964 (D. Haig et al., J. Comp. Pathol. 1964, 74, 81-84). Depuis cette date, 5 sérotypes d'adénovirus porcins ont été identifiés et décrits (Hirahara et al., J. Vet. Sci. 1990, 52, 407-409). Les adénovirus des sérotypes 1 et 2 sont associés à des diarrhées ; les adénovirus du sérotype 4 sont associés à des symptômes de pneumonie et d'encéphalite. Des adénovirus porcins ont été classés dans un cinquième sérotype du fait de leur absence de neutralisation croisée avec des antisérums spécifiques des PAV des sérotypes 1 à 4. Le génome de PAV-5 a été analysé et une carte de restriction a été établie (Tuboly *et al.,* Virus Res. 1995, **37**, 49-54). Cette analyse a montré l'absence de similitude entre les cartes de restriction des PAV-5 et celles des PAV des autres sérotypes : PAV-1 et PAV-2 (Reddy *et al.,* Arch. Virol. 1995, **140**, 195-200), PAV-3 (Reddy *et al.,* Intervirology 1993, **36**, 161-168), et PAV-4 (Kleiboeker *et al.,* Arch. Virol. 1993, **133**, 357-368). Parmi les sérotypes 3 et 5, il existe des souches naturellement non pathogènes pour le porc.

Les PAV ont un génome d'une taille d'environ 32 à environ 34 kpb. Le génome de PAV-1 a une taille d'environ 33,5 kpb. Celui de PAV-2 a une taille d'environ 33,3 kpb. La séquence complète du virus PAV-3 a été récemment établie et déposée dans la banque de données GenBank, sous le numéro AF083132 (P. S. Reddy *et al.,* Virol. 1998, **251**, 414-426) ; sa taille est de 34094 paires de bases (pb). Le génome de PAV-4 a une taille d'environ 32 kpb et celui de PAV-5 a une taille d'environ 33,2 kpb. Le génome complet de PAV-5 et, notamment la région E3, n'a pas été séquencé et publié.

L'application principale envisagée pour les adénovirus se trouve dans le domaine de la thérapie génique chez l'homme et un très grand nombre de constructions et de systèmes ont été proposés. Les adénovirus ont également été proposés comme vecteurs recombinés dans des compositions vaccinales pour la protection des humains et des animaux contre de nombreux virus pathogènes.

A ce jour, deux stratégies de construction de vecteurs adénovirus recombinés ont été développées (M. Eloit & M. Adam, J. Gen. Virol. 1995, **76**, 1583-1589).

La première stratégie consiste en l'insertion de cassettes d'expression dans des régions indispensables à la réplication de l'adénovirus, d'où la nécessité de développer en parallèle des systèmes de transcomplémentation pour pouvoir assurer la réplication du virus. Pour les adénovirus porcins, la région E1 a été proposée comme site d'insertion (P. S. Reddy *et al*., Virus Res. 1998, **58**, 97-106). Les vecteurs recombinés ainsi construits sont dits " non réplicatifs " car ils ne peuvent pas se répliquer chez l'hôte auquel ils sont administrés.

En variante de cette première stratégie, on peut utiliser un virus non réplicatif chez l'animal destinataire. La cassette d'expression PRV gD a notamment été déjà insérée dans le site E1A de l'adénovirus humain de sérotype 5, non réplicatif chez le porc (M. Monteil *et al*., J. Gen. Virol. 1997, **78,** 3303-3310 ; A. Ambriovic *et al*., Virol. 1997, **238,** 327-335).

La seconde stratégie consiste en l'insertion de cassettes d'expression au niveau de régions du génome viral qui sont non essentielles à la réplication de l'adénovirus. La difficulté de déterminer des régions non essentielles chez les adénovirus et la présence d'une capside rigide rendent cette seconde stratégie difficile à mettre en oeuvre.

En effet l'une des caractéristiques essentielles des adénovirus est qu'ils possèdent une capside rigide qui limite strictement la taille de la molécule d'ADN pouvant y être encapsidée. De manière générale, on estime qu'il est possible d'encapsider une molécule d'ADN correspondant à 105 à 114% de la taille du génome, ce qui correspond, selon la taille des génomes, à des inserts d'environ 1,7 à environ 3,9 kpb. En cas de tailles excédentaires, des délétions et/ou des réarrangements non voulus au sein du génome recombiné peuvent se produire.

La région E3 n'est conservée ni en taille, ni en organisation génétique d'un adénovirus d'une espèce donnée à un adénovirus d'une autre espèce, ni entre les adénovirus porcins des différents sérotypes (S. Kleiboeker, Virus Res. 1994, **31**,17-25).

La région E3 du virus PAV-3, a une taille de 1179 paires de bases. Il s'agit d'une région complexe, qui code pour au moins 3 Cadres Ouverts de Lecture (COL). Ces COL se chevauchent entre eux, et le premier COL chevauche aussi en partie le gène codant pour la protéine pVIII (P. S. Reddy *et al.,* Virus Res. 1995, **36**, 97-106), qui est une protéine essentielle.

La présence de ces COL chevauchants dans la région E3 représente une difficulté majeure pour y déterminer les sites d'insertions et/ou les limites des délétions de manière que les adénovirus recombinés restent réplicatifs chez les porcins.

La séquence en acides aminés codée par le second COL de la région E3 ne montre aucune homologie entre les adénovirus PAV-3 et HAV-2 (humain, de sérotype 2), ni avec aucune protéine d'adénovirus actuellement connue (P. S. Reddy *et al*., Virus Res. 1995, **36**, 97-106). Les séquences en acides aminés prédites d'après la séquence nucléotidique du premier COL de la région E3 chez PAV-3 n'ont que 33,3% d'identité avec celle de l'adénovirus canin de sérotype 2 (CAV-2). Non seulement les protéines codées par des régions E3 de différents adénovirus ne sont pas homologues, mais aussi celles codées par les COL de la région E3 ne montrent aucune homologie entre les adénovirus PAV-3 et PAV-4 (S. B. Kleiboeker, Virus Res. 1994, **31**, 17-25).

La région E3 a une taille de 1162 pb chez PAV-1 (avec 5 COL) ; elle est de 1222 pb chez PAV-2 (avec 5 COL) (P. S. Reddy *et al*., Virus Res. 1996, **43**, 99-109), de 1879 pb chez PAV-4 (avec 6 COL). Seul le quatrième COL de la région E3 du virus PAV-4 (correspondant à une protéine de 13,2 kDa) présente une homologie avec un autre COL connu de la région E3, codant pour la protéine 14,7 kDa de l'adénovirus humain de type 5 (en anglais Human AdenoVirus 5 ou HAV-5). La région E3 de PAV-4 ne présente aucune homologie de séquence avec les régions E3 des autres adénovirus porcins, et notamment avec celles de PAV-3 et de PAV-5.

Outre la difficulté de définition de leurs limites pour conserver le caractère réplicatif *in vivo,* les délétions dans la région E3 ne sont pas non plus sans risque. Les délétions dans la région E3 peuvent avoir en effet des conséquences sur la pathogénicité des adénovirus recombinés. Ainsi, il a été observé qu'une délétion dans la région E3 du virus HAV-5 augmentait la pathogénicité pulmonaire de ce virus dans le modèle expérimental d'infection du rat coton (Ginsberg *et al.,* Proc. Natl. Acad. Sci. USA 1989, **86**, 3823-3827).

Ceci montre que les conséquences des délétions dans la région E3 d'un adénovirus doivent être considérées au cas par cas, et non par simple transposition des observations faites sur un adénovirus particulier vers un autre. L'organisation génétique de la région E3 de PAV-3 est unique, celle de la région E3 de PAV-5 également.

La localisation précise d'un site d'insertion dans la région E3 se complique encore du fait de la présence de zones d'épissage et de polyadénylation complexes, zones caractéristiques des adénovirus (imperiale *et al.,* Curr. Top. Microbiol. Immunol. 1995, **199**, 139-171). Les zones d'épissage localisées dans la région E3 sont importantes pour la maturation de nombreux ARN messagers essentiels codés par les séquences localisées hors de la région E3. La région E3 est elle-même située dans une partie du génome viral à haute activité transcriptionnelle (P. Sharp, 1984, in The Adenovirus, Ed. H. S. Ginsberg, Plenun Press, New York and London, 173-204), l'insertion d'une séquence nucléotidique hétérologue dans la région E3 peut avoir des impacts négatifs sur l'activité biologique du virus recombiné. De plus, la région E3 est localisée an aval du promoteur tardif majeur (major late promoter, MLP), région où des interférences entre la transcription de l'insert et celle initiée par le promoteur MLP ont été démontrées (Xu *et al.;* J. Gen. Virol. 1995, **76**, 1971-1980).

Les résultats obtenus avec des adénovirus provenant d'autres espèces (humain, bovin, ovin...) ne sont donc pas transposables aux adénovirus porcins. Aucun vecteur recombiné réplicatif n'a ainsi été produit à ce jour à partir d'un adénovirus porcin.

La déposante s'est donnée pour objectif de rendre accessible l'insertion de gènes hétérologues dans le génome des virus PAV-3 et PAV-5 sans que leur capacité de réplication *in vivo* n'en soit modifiée de manière substantielle.

Un autre objectif de l'invention est de proposer des régions d'insertion susceptibles d'être délétées sans remettre en cause cette capacité de réplication, de manière à accroître la capacité d'insertion dans le virus.

Un autre objectif encore de l'invention est de proposer des virus PAV-3 recombinés conservant une capacité de réplication *in vivo* et permettant leur usage en tant que vaccins vivants recombinés, y compris vaccins administrés par voie muqueuse et/ou en présence d'anticorps d'origine matemelle.

La déposante a réussi à modifier la région E3 de PAV-3 et PAV-5 tout en conservant la capacité de réplication *in vivo.* Elle a également pu démontrer l'expression *in vivo* d'un gène inséré dans cette région. Elle rend donc accessible l'utilisation de PAV-3 et PAV-5 comme vecteurs d'expression réplicatifs.

La présente invention a donc pour objet un adénovirus porcin de sérotype 3 comprenant au moins une séquence nucléotidique hétérologue insérée dans le génome de PAV-3 dans des conditions permettant aux virus recombinés obtenus de se répliquer *in vivo* chez les porcins et d'exprimer la séquence insérée. Ces virus modifiés présentent des avantages importants en matière de vaccination et notamment : ils sont efficaces même en présence d'anticorps maternels et peuvent être alors efficacement utilisés par voie muqueuse.

De manière préférée, on insère une séquence hétérologue dans une zone non essentielle du génome, notamment de la région E3, avec une délétion de cette zone d'insertion. Par délétion d'une zone, on entend une délétion totale ou partielle, de préférence totale, de la zone d'insertion. En d'autres termes, la séquence hétérologue est insérée à la place de tout ou partie de la zone d'insertion, de préférence à la place de la totalité de cette zone.

Pour le PAV-3, la zone d'insertion préférée dans E3 est une zone comprenant tout ou partie des nucléotides 706 à 1624 de la séquence publiée par P.S. Reddy et al., Virus Research 1995. 36.97-106, ce qui correspond aux positions 27 794 à 28 712 de la séquence publiée dans GenBank AN # U10433. En d'autres termes, cette zone d'insertion et son éventuelle délétion peut comprendre la totalité ou une partie seulement de la séquence 706 à 1624 et/ou s'étendre dans E3 au delà de la limite 706 et/ou de la limite 1624 et éventuellement comprendre E3 entière. De préférence, cette zone est constituée de, ou comprend, E3, les nucléotides 706 à 1624, les nucléotides 1002 à 1624 ou encore tout ou partie de la séquence 706 à 1002. Elle comprend notamment au moins la séquence 1002 à 1624.

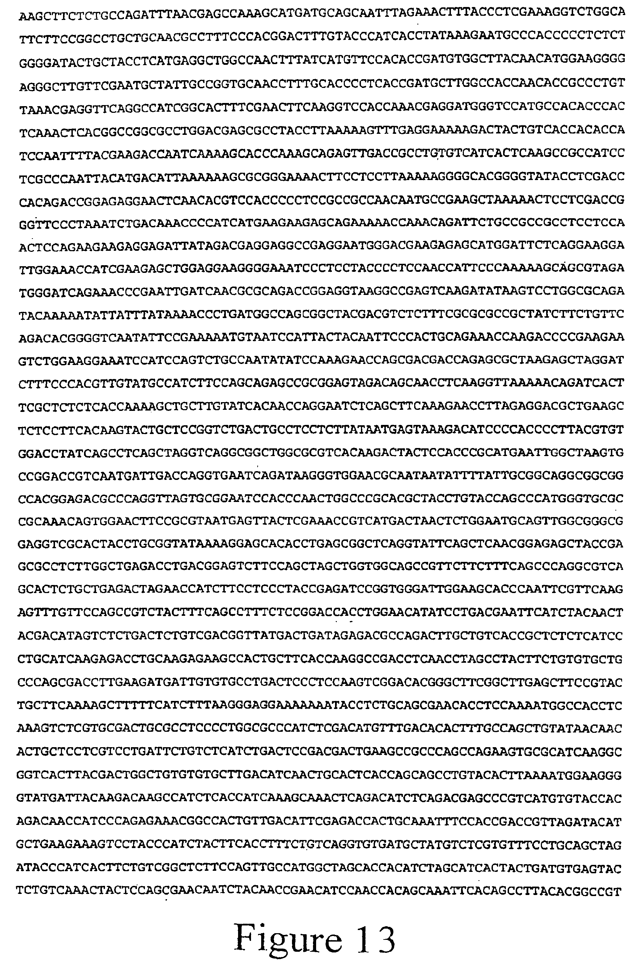

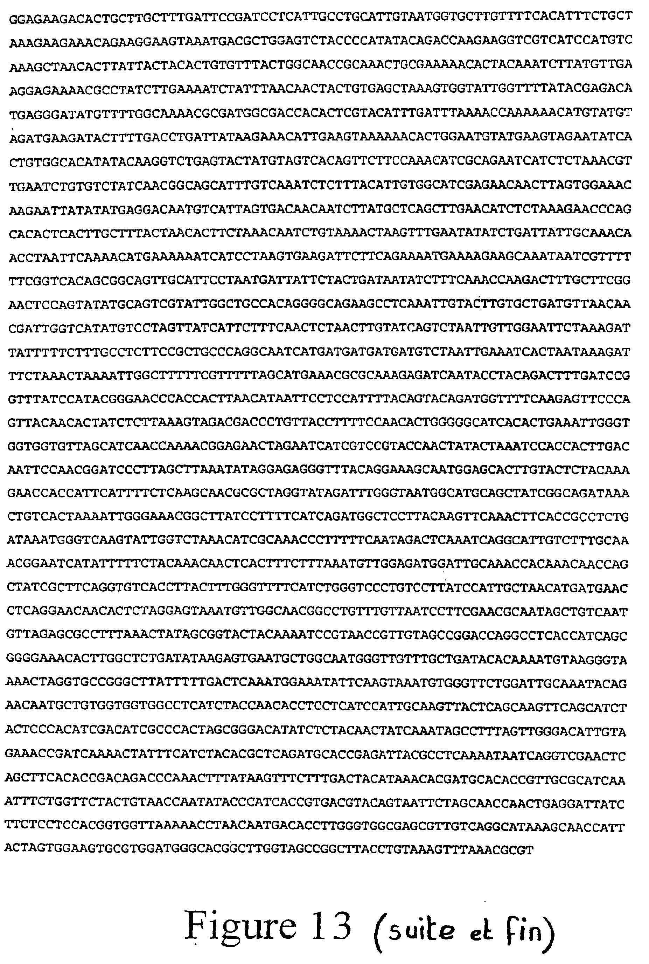

Pour le PAV-5, E3 est définie par la séquence de nucléotides 2382 à 4042 à la SEQ ID NO : 5. La zone d'insertion et son éventuelle délétion inclut E3 et comprend tout ou partie des nucléotides 2064 à 4083, par exemple tout ou partie des nucléotides 2389 à 3861, de la SEQ ID NO : 5 (figure 13). En d'autres termes, cette zone d'insertion et son éventuelle délétion peut comprendre une partie seulement de la séquence 2389 à 3861 et/ou s'étendre dans E3 au delà de la limite 2389 et/ou de la limite 3861 et éventuellement comprendre E3 entière. Lorsque l'on délète jusqu'au nucléotide 4083 inclus, on préfère alors insérer à la place du nucléotide 4083 un site accepteur d'épissage. De préférence, cette zone d'insertion est constituée essentiellement par les nucléotides 2389 à 3861.

L'invention entend bien sûr couvrir aussi l'insertion dans les zones correspondantes des autres souches de PAV-3 dont les séquences diffèrent de celles des souches de référence selon l'invention.

La présente invention a également pour objet un virus PAV-3 ayant une capacité d'insertion supérieure en taille, tout en restant réplicatif *in vivo* chez les porcs. Le maintien de la propriété de réplication chez l'hôte est recherché afin d'obtenir une efficacité de protection optimale, en particulier en utilisant une composition vaccinale par voie muqueuse et/ou en présence d'anticorps d'origine maternelle

L'augmentation de la capacité d'insertion se fait au moyen d'une délétion, e.g. de tout ou partie de la région E3, la partie de E3 pouvant être délétée étant située entre le gène codant pour la protéine pVIII et celui codant pour la fibre. Notamment, la présente invention décrit des délétions de 622 pb et 919 pb qui peuvent être réalisées dans la région E3 du virus PAV-3, tout en conservant le caractère réplicatif des virus recombinés (voir exemples 6 à 10). Une délétion de 1472 pb dans la région E3 du virus PAV-5, aboutissant aux mêmes caractéristiques phénotypiques du virus recombiné (voir exemples 14 à 17) est décrite.

L'adénovirus PAV-3 recombiné obtenu selon l'invention peut avantageusement servir à une insertion d'une taille maximale d'environ 3,0 kpb pour le virus PAV-3 et à son expression chez le porc.

L'invention a donc aussi pour objet le vecteur PAV-3 délété, notamment dans la région E3, tout en restant réplicatif, en particulier délété dans les zones sus-mentionnées, c'est-à-dire délété de tout ou partie de la zone considérée, de préférence de la totalité de cette zone.

Les séquences nucléotidiques hétérologues pouvant être insérées sont des séquences codant pour des immunogènes ou des fragments, e.g. épitopes, immunologiquement actifs d'immunogènes, et notamment des gènes ou des fragments de gènes (e.g. épitopes) codant pour des immunogènes de pathogènes porcins.

Dans le cadre de la présente invention, on peut bien entendu insérer plus d'une séquence hétérologue dans le même virus. On peut notamment y insérer des séquences hétérologues provenant d'un même virus ou de virus différents.

Selon une modalité particulière, il est possible d'insérer des séquences artificielles regroupant plusieurs fragments ou épitopes provenant d'un même pathogène ou de pathogènes différents, et appelées "séquences poly-épitopes". Dans ce dernier cas, la séquence poly-épitopes est placée sous la dépendance d'un promoteur unique.

On peut également insérer des séquences codant pour des immunomodulateurs, en particulier des cytokines, de préférence des cytokines de porc telles que le "Granulocyte-Macrophage Colony Stimulating Factor" porcin (poGM-CSF), l'interleukine porcine 4 (poiL-4), l'IL-12 porcine ou l'IL-18 porcine.

L'expression de plusieurs gènes hétérologues insérés dans le locus d'insertion peut également être rendue possible par insertion d'une séquence appelée "IRES" (Internal Ribosome Entry Site) provenant notamment d'un picomavirus tel que le virus de la maladie vésiculaire du porc (swine vesicular disease virus, SVDV ; B.-F. Chen *et al.,* J. Virology, 1993. **67,** 2142-2148), le virus de l'encéphalomyocardite (EMCV ; R.J. Kaufman *et al.,* Nucleic Acids Research, 1991. **19**, 4485-4490), le virus de la fièvre aphteuse (FMDV ; N. Luz et E. Beck, J. Virology, 1991. **65**, 6486-6494), ou encore d'une autre origine. L'homme du métier pourra se reporter au contenu des 3 articles cités. La cassette d'expression de deux gènes aurait donc la structure minimale suivante : promoteur (optionnel) - gène 1 - IRES - gène 2 - signal de polyadénylation. Le vaccin vivant recombiné selon l'invention pourra donc comprendre, insérée dans le locus d'insertion, une cassette d'expression comprenant successivement un promoteur, deux ou plusieurs gènes séparés deux à deux par un IRES, et un signal de polyadénylation.

Les pathogènes porcins sont des virus, des bactéries, des parasites, notamment choisis parmi ceux du groupe consistant en virus de la maladie d'Aujeszky (virus PRV ou pseudorage), virus de la grippe porcine (virus influenza porcin, SIV), virus de la maladie mystérieuse du porc (virus PRRS), virus de la parvovirose (virus PPV), virus de la peste porcine classique (virus HCV ou Hog Cholera Virus), circovirus porcin, notamment de type 2 (Porcine CircoVirus type 2, PCV2 ou virus du syndrome de dépérissement généralisé du porcelet), *Actinobacillus pleuropneumoniae, Mycoplasma hyopneumoniae.*

En ce qui concerne la valence Aujeszky, on peut mettre en oeuvre les gènes gB (Robbins A.K. *et al.* J. Virol. 1987. **61**. 2691-2701 GenBank AN # M17321), gC (Ishikawa K. *et al.* Vet. Microbiol. 1996. **49**. 267-272 GenBank AN # D49435) et/ou gD (Rivière M. *et al*. J. Virol. 1992. **66**. 3424-3434 et Hong W.Z. *et al*. GenBank AN # AF086702), sous leurs formes natives ou sécrétées.

En ce qui concerne la valence grippe porcine, on préfère mettre en oeuvre les gènes HA (WO-A-98/03658 exemples 10 et 12) (formes native ou sécrétée), NA (Nerome K. *et al.* J. Gen Virol. 1995. **76.** 613-624 GenBank AN # D21194) (formes native ou sécrétée) et/ou NP (WO-A-98/03658 exemples 11 et 13).

En ce qui concerne la valence PRRS, on préfère utiliser les gènes ORF₄, ORF3 (formes native ou sécrétée), ORF5 (formes native ou sécrétée), ORF6 et/ou ORF7 de souches européennes (Meulenberg J. *et al*. Virology. 1993. **192.** 62-72) et de souches américaines (Mardassi H. *et al*. Arch. Virol. 1995. **140.** 1405-1418).

En ce qui concerne la valence peste porcine classique, on préfère utiliser les gènes EO, E1 (formes native ou sécrétée) et/ou E2 (formes native ou sécrétée) (Meyers G. *et al.* Virology. 1989. **171.** 18-27).

En ce qui concerne la valence parvovirose, on préfère utiliser le gène de capside VP2 (Vasudevacharya J. *et al.* Virology. 1990**. 178.** 611-616).

En ce qui concerne la valence PCV2, on préfère utiliser les gènes ORF1 et/ou ORF2, mais de préférence ORF1 et ORF2 (Meehan B. *et al.* J. Gen. Virol. 1998. **79.** 2171-2179)**.**

Les séquences hétérologues sont insérées sous la dépendance de signaux régulateurs de la transcription et notamment d'un promoteur, de préférence apportés lors de la recombinaison. Il n'est toutefois pas exclu de faire exprimer ces séquences hétérologues sous le contrôle de signaux propres à l'adénovirus servant de vecteur.

Parmi les promoteurs utiles, on préfère utiliser des promoteurs eucaryotes forts, tels que le LTR du virus du sarcome de Rous et de préférence le promoteur précoce du cytomegalovirus ou CMV-IE (CytoMegaloVirus Immediate Early), notamment d'origine murine (MCMV-IE) ou humaine (HCMV-IE), ou encore ceux d'autres origines e.g. du porc, du singe, du rat ou de cobaye.

De manière particulièrement avantageuse, on peut utiliser les promoteurs CMV-IE avec une partie activatrice (en anglais "enhancer") (US-A-5.168.062, US-A-4.968.615, US-A-4.963.481). Il peut être avantageux de recourir à des fragments de ces promoteurs, conservant l'activité promotrice, de préférence avec la partie activatrice, e.g. les promoteurs CMV-IE tronqués selon WO-A-98/00166. L'homme du métier pourra se reporter pour plus de détails à WO-A-98/00166, et notamment son exemple 12.

La présente invention a aussi pour objet les préparations immunogènes ou immunologiques et les vaccins porcins comprenant un adénovirus recombiné conforme à l'invention, dans un véhicule ou excipient acceptable sur le plan vétérinaire, et éventuellement un adjuvant. Par définition, une préparation immunogène ou immunologique induit au moins une réponse immunitaire (cellulaire et/ou humorale) après administration chez l'animal. Un vaccin induit une réponse protectrice.

Les adjuvants sont de préférence choisis parmi les polymères de l'acide acrylique ou méthacrylique et les copolymères d'anhydride maléique et de dérivé alcényle.

Les composés préférés sont les polymères de l'acide acrylique ou méthacrylique réticulés, notamment par des éthers polyalcényliques de sucres ou de polyalcools. Ces composés sont connus sous le terme carbomère (Pharmeuropa vol. 8, n° 2, juin 1996). L'homme de l'art peut aussi se référer à US-A-2 909 462 décrivant de tels polymères acryliques réticulés par un composé polyhydroxylé ayant au moins 3 groupes hydroxyle, de préférence pas plus de 8, les atomes d'hydrogène d'au moins trois hydroxyles étant remplacés par des radicaux aliphatiques insaturés ayant au moins 2 atomes de carbone. Les radicaux préférés sont ceux contenant de 2 à 4 atomes de carbone, e.g. vinyles, allyles et autres groupes éthyléniquement insaturés. Les radicaux insaturés peuvent eux-mêmes contenir d'autres substituants, tel que méthyl. Les produits vendus sous la dénomination Carbopol® (BF Goodrich, Ohio, USA) sont particulièrement appropriés. Ils sont réticulés par un allyl saccharose ou par de l'allylpentaérythritol. Parmi eux, on peut citer les Carbopol® 974P, 934P et 971 P.

Parmi les copolymères d'anhydride maléique et de dérivé alcényle, on préfère les EMA® (Monsanto) qui sont des copolymères d'anhydride maléique et d'éthylène, linéaires ou réticulés, par exemple réticulés par du divinyléther. On peut se référer à J. Fields *et al.,* Nature, **186** : 778-780, 4 juin 1960.

Sur le plan de leur structure, les polymères d'acides acrylique ou méthacrylique et les EMA® sont formés de préférence de motifs de base de formule suivante : 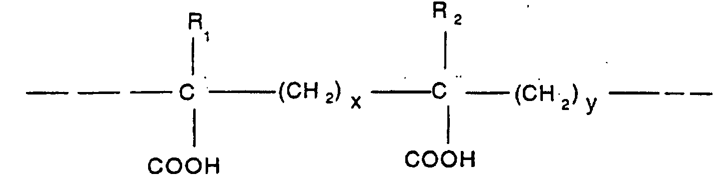
dans laquelle:
- R₁ et R₂, identiques ou différents, représentent H ou CH₃
- X = 0 ou 1, de préférence x = 1
- Y=1 1 ou 2, avec x + y = 2

Pour les EMA®, x = 0 et y = 2. Pour les carbomères, x = y = 1.

La dissolution de ces polymères dans l'eau conduit à une solution acide qui sera neutralisée, de préférence jusqu'à pH physiologique, pour donner la solution adjuvante dans laquelle le vaccin proprement dit sera incorporé. Les groupes carboxyliques du polymère sont alors en partie sous forme COO⁻.

De manière préférée, on réalise une solution d'adjuvant selon l'invention, notamment de carbomère, dans de l'eau distillée, de préférence en présence de chlorure de sodium, la solution obtenue étant à pH acide. On dilue cette solution mère en l'ajoutant dans la quantité nécessaire (pour l'obtention de la concentration finale souhaitée), ou une partie importante de celle-ci, d'eau chargée en NaCl, de préférence eau physiologique (NaCl 6 g/l), en une ou plusieurs fois avec neutralisation concomitante ou subséquente (pH 7,3 à 7,4), de préférence par NaOH. Cette solution à pH physiologique sera utilisée telle quelle pour reprendre le vaccin, notamment conservé sous forme lyophilisée.

La concentration en polymère dans la composition vaccinale finale sera de 0.01 % à 2 % P/V, plus particulièrement de 0.06 à 1 % P/V, de préférence de 0,1 à 0,6 % P/V.

Les préparations immunogènes et vaccins selon l'invention peuvent comprendre plusieurs adénovirus recombinés conformes à l'invention, comprenant des séquences nucléotidiques hétérologues différentes, provenant de pathogènes identiques et/ou différents.

La présente invention a aussi pour objet les méthodes d'immunisation et de vaccination des porcs contre un ou plusieurs pathogènes porcins, comprenant l'administration de doses efficaces des préparations immunogènes et vaccins ci-dessus. Les doses seront par exemple de 10⁴ à 10⁷ DICC50.

Elle vise notamment l'administration de ces préparations immunogènes et vaccins par voie muqueuse, e.g. orale, nasale, oculaire, et/ou à de jeunes animaux présentant des anticorps maternels. Les autres voies classiquement employées pour la vaccination des porcs (intramusculaire en particulier, intradermique...) peuvent être aussi utilisées.

La présente invention a également pour objet des méthodes d'obtention de vecteurs recombinés PAV-3, des préparations immunogènes et des vaccins les incorporant.

Elle a aussi pour objet l'utilisation de ces vecteurs pour la fabrication des préparations immunogènes et des vaccins selon l'invention, notamment destinés à une administration par voie muqueuse et/ou à des jeunes animaux présentant des anticorps maternels et/ou à une administration par les voies classiques.

L'invention va être maintenant décrite plus en détail à l'aide de modes de réalisation pris à titre d'exemples non limitatifs et se référant au dessin dans lequel :

### Liste des figures :

- Figure 1 :: Schéma des plasmides pKS-Droit ITR3, pPolylI-Droit ITR3 et pITRsPAV3
- Figure 2 :: Schéma des plasmides pE3PAV3, pE3dI622CMV-EGFP et pE3dI622CMV-gD
- Figure 3 :: Carte de restriction des plasmides pE3PAV3 et pE3dI919
- Figure 4 :: Schéma des plasmides pCMV-EGFP, pCMV-gD et pgD
- Figure 5 :: Carte de restriction des plasmides pCMV-EGFP, pgD, pE3dI919CMV-EGFP et pE3dI919CMV-gD
- Figure 6 :: Carte de restriction des plasmides pE3dI622EGFP et pE3dI919EGFP
- Figure 7 :: Carte de restriction des plasmides pE3dl622gD et pE3dI919CMV-gD
- Figure 8 :: Carte de restriction des plasmides pCR-Droit ITR5, pCR-Gauche ITR5, pPolyll-Droit ITR5 et pITR-PAV5
- Figure 9 :: Carte de restriction du plasmide pE3PAV5
- Figure 10 :: Carte de restriction des plasmides pE3dI1472CMV-EGFP et pE3dI1472CMV-gD
- Figure 11 :: Carte de restriction des plasmides pE3dl1472EGFP et pE3dl1472gD
- Figure 12 :: Carte physique de PAV-5, digestion BamHl
- Figure 13 :: Séquence de 5614 paires de bases (région E3 et séquences adjacentes du virus PAV-5)

### Liste des séquences :

- SEQ ID NO 1 :: Oligonucléotide ITRPAV3
- SEQ ID NO 2 :: Oligonucléotide T3
- SEQ ID NO 3 :: Oligonucléotide T7
- SEQ ID NO 4 :: Oligonucléotide ITRPAV5
- SEQ ID NO 5 :: Séquence de la région E3 du virus PAV-5

### Exemples:

### Exemple 1 : Cellules et virus

Les milieux de culture de cellules et les réactifs ont été fournis par Gibco BRL. Les milieux (Dulbecco's MEM avec Glutamax-1 Cat # 61965-026) ont été additionnés de 1 mM de pyruvate de sodium (Cat # 11360-039), de gentamycine (50µg/ml, Cat # 15710-031) et de 10% de sérum de veau foetal (Cat # 10270-106 lot 40Q5774K).

Les cellules PK-15 (N° ATCC CCL33) et ST (N° ATCC CRL 1756) ont été utilisées pour la culture des virus.

Le virus sauvage adénovirus porcin de type 3 (= PAV-3), non pathogène chez le porc, a été obtenu du laboratoire du Dr. Eva Nagy (Université de Guelph, Service de Pathobiologie, 50 Stone Road Guelph, ONTARIO, Canada, NIG 2W1) (Reddy P. *et al.* Virus Res. 1996. **43**. 99-109).

Le virus sauvage adénovirus porcin de type 5 (= PAV-5), non pathogène chez le porc, a été obtenu du laboratoire du Dr. Tadashi Hirahara (Kyoto Biken Laboratories Inc., Division de Microbiologie Vétérinaire, 24-16 Makishima-cho, Ujishi, KYOTO, 611 Japon) (Hirahara T. *et al.* J. Vet. Sci. 1990. **52**. 407-409).

Conditions de culture et de passages : deux fois par semaine, les cellules sont trypsinées (solution de trypsine à 2,5% (Sigma Cat # 044-5075) diluée 50 fois en solution saline équilibrée de Earle (Gibco-BRL Cat # 14155-030)) et resuspendues dans du milieu de culture (dilution 1:6). Les cellules sont cultivées à +37°C en présence de 5% de CO2 avant d'être remises en culture.

### Exemple 2 : Enzymes, bactéries, plasmides et techniques de biologie moléculaire

Les enzymes de restriction, la Taq polymérase pour les réactions d'amplification en chaîne, et les autres enzymes nécessaires aux différentes modifications des ADN ont été fournies respectivement par New England Biolabs Inc., Roche Diagnostics, et Gibco-BRL.

Toutes ces enzymes ont été utilisées selon les recommandations des fournisseurs. Les techniques standards de biologie moléculaire ont été réalisées comme décrites dans " Molecular Biology : A Laboratory Manual". 2nd edition. (Sambrook *et al*. Cold Spring Harbor Laboratory, New York. 1989).

Les bactéries compétentes BJ5183 (Hanahan D. J. Mol. Biol. 1983. **166**. 557-580) ont été préparées comme suit :

60 ml d'une culture de ces bactéries en phase exponentielle ont été préparés selon la technique du chlorure de calcium (Sambrook *et al.* 1989). Elles ont été récoltées dans un volume final de 2 ml de CaCl2 50 mM. 300 µl de cette suspension de bactéries ont été mélangés avec les différents ADN partenaires de recombinaison homologue contenus dans un volume de 100 µl de Tris-HCI 100 mM pH 7.4, laissées en contact pendant 30 mn sur glace fondante, puis les bactéries ont été traitées par un choc thermique à +45°C pendant 2 minutes, laissées au repos sur glace fondante pendant 10 minutes, et enfin étalées sur un milieu sélectif.

### Exemple 3 : Extraction des ADN viraux de PAV-3 et de PAV-5

Les virus sont cultivés et amplifiés en flacons Falcon de 10 cm² selon les conditions de l'exemple 1. Lorsque l'ECP est complet, les cellules sont récoltées, lysées par 3 cycles de congélation/décongélation, puis la suspension virale est clarifiée par centrifugation à basse vitesse. Les virus PAV-3 et PAV-5 sont purifiés sur gradient de chlorure de césium (1,34 g/ml) et la bande virale est récoltée, purifiée, puis les ADN viraux sont obtenus après traitement à la protéinase K et extraction au phénol/chloroforme (Sambrook *et al.* 1989). Ces ADN ont ensuite été utilisés pour les différentes étapes de clonage ou d'amplification en chaîne par polymérase (ACP).

### Exemple 4 : Construction du plasmide "pITRs PAV3"

L'ADN génomique du virus PAV-3, préparé selon l'exemple 3, a été digéré par EcoRI et le fragment EcoRI D de 245 pb a été isolé après électrophorèse en gel d'agarose. Ce fragment a été ensuite ligaturé avec le plasmide pBlueScript KS (pBS-KS) (Stratagene Inc. La Jolla, CA) (GenBank # X52327), préalablement digéré par EcoRI et déphosphorylé, pour donner le plasmide identifié "pKS-EcoDPAV3".

Une réaction d'ACP a été effectuée avec la matrice du plasmide pKS-EcoDPAV3 et avec les oligonucléotides suivants :
ITRPAV3 (SEQ ID N°1) (50 mer) : et
T3 (SEQ ID N°2) (20 mer) :

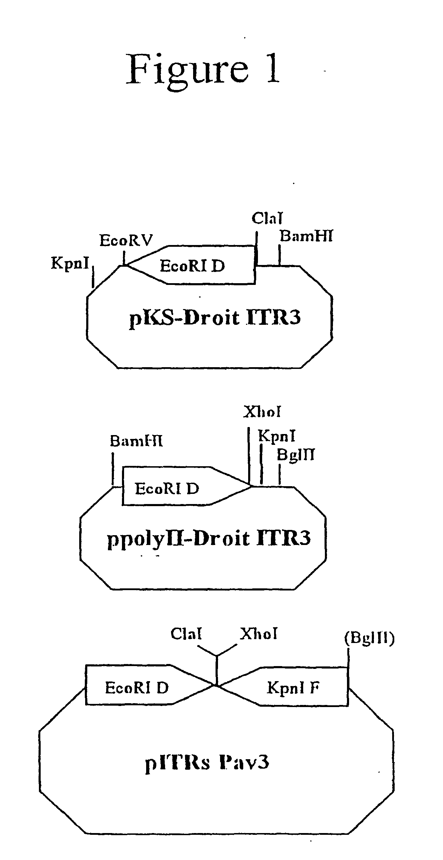

Le produit d'amplification (367 pb) a été traité avec l'ADN polymérase T4 pour le rendre "bouts francs" , puis il a été digéré avec l'enzyme Clal, et ligaturé avec le plasmide pBS-KS, préalablement digéré par Clal et EcoRV, pour donner le plasmide identifié "pKS-Droit ITR3" (figure 1).

Le fragment EcoRI D a alors été libéré du plasmide pKS-Droit ITR3 par digestion avec les enzymes BamHI et KpnI, et le fragment de restriction BamHI-KpnI de 353 pb a été ligaturé avec le plasmide pPolyll (Lathe R. *et al.* Gene. 1987. **57**. 193-201) (GenBank # G209113), préalablement digéré par BamHI et Kpnl, pour générer le plasmide identifié "pPolyll-Droit ITR3" (figure 1).

L'ADN génomique du virus PAV-3, préparé selon l'exemple 3, a été digéré par Kpnl pour isoler, après électrophorèse en gel d'agarose, le fragment terminal Kpnl F de 939 pb. Ce fragment a été ligaturé avec le plasmide pBS-KS, préalablement digéré par Kpnl et déphosphorylé, pour donner le plasmide identifié "pKS-KpnFPAV3" .

Une réaction d'ACP a ensuite été effectuée avec la matrice du plasmide pKS-KpnFPAV3 et avec les oligonucléotides suivants :
ITRPAV3 (SEQ ID N°1) (50 mer) : et
T7 (SEQ ID N°3) :

Le produit d'amplification (1086 pb) a été traité avec l'ADN polymérase T4 pour le rendre "bouts francs", puis il a été digéré avec l'enzyme XhoI, et ligaturé avec le plasmide pPolyll-Droit ITR3, préalablement digéré par BgIII, traité par l'ADN polymérase T4, puis digéré par XhoI, pour générer le plasmide identifié "pITRsPAV3" (figure 1).

### Exemple 5 : Construction du plasmide pPAV3

Le plasmide pITRsPAV3 (exemple 4) a été linéarisé par digestion avec ClaI, puis co-transformé dans des bactéries compétentes BJ5183 avec l'ADN génomique du virus PAV-3 préparé selon l'exemple 2. Cette co-transformation a permis de générer, par recombinaison homologue dans *Escherichia coli* (Chartier C. *et al.* J*.* Virol. 1996. **70**. 4805-4810), un plasmide contenant le génome complet cloné de PAV-3. Ce plasmide a été désigné "pPAV3".

### Exemple 6 : Construction des plasmides navettes PAV-3

### 6.1. Construction du plasmide avec une délétion de 622 paires de bases (petite délétion) dans la région E3

Le plasmide pNEB193 (New England BioLabs Cat # 305-1) a été digéré par PacI, traité avec l'ADN polymérase T4, puis religaturé sur lui-même pour générer le plasmide "pNEBPac-".

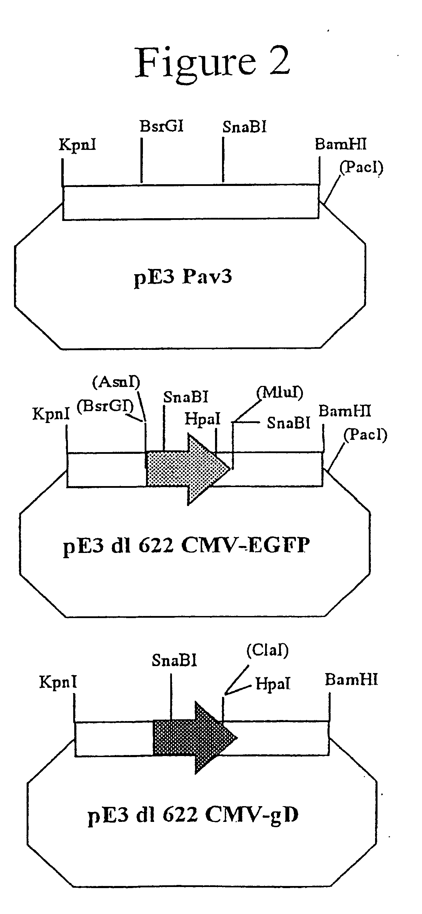

Le plasmide pPAV3 (exemple 5) a été digéré par Kpnl et BamHI pour isoler le fragment KpnI-BamHI de 4344 pb contenant la région E3 (Reddy *et al*. Virology. 1998. **251.** 414-426). Ce fragment a été ligaturé avec le plasmide pNEBPac-, préalablement digéré par Kpnl et BamHI, pour donner le plasmide "pE3PAV3" (figure 2). La séquence du fragment KpnI-BamHI a été vérifiée et a été trouvée identique à la séquence publiée par Reddy P. *et al*. (Virus Research. 1995. **36.** 97-106 et Virology. 1998. **251.** 414-426) (GenBank AN # U10433 et AN # AF083132). L'homme du métier pourra se reporter à ces références.

Le plasmide pEGFP-F (Chalfie M. *et al.* Science. 1994. **263.** 802-805 ; Clontech Cat # 6074-1) a été modifié pour éliminer complètement le polylinker. Cette délétion a été obtenue par une digestion avec BamHI, suivie d'un traitement par l'ADN polymérase T4. La religature sur lui-même du vecteur ainsi modifié a généré le plasmide pCMV-EGFP. Le plasmide pCMV-EGFP a été ensuite digéré par les enzymes Asnl et Mlul, puis traité avec l'ADN polymérase T4, pour isoler le fragment Asnl (bout franc) - Mlul (bout franc) de 1,6 kpb (= cassette d'expression CMV-EGFP). Ce fragment a été ligaturé avec le plasmide pE3PAV3, préalablement digéré par BsrGI et SnaBI et traité avec l'ADN polymérase T4, pour générer le plasmide pE3dl622CMV-EGFP (figure 2). La délétion créée entre les sites SsrGI et SnaBI de la région E3 du virus PAV-3 a une taille de 622 paires de bases. Cette délétion s'étend des nucléotides 1002 à 1624 de la séquence publiée par P. Reddy *et al.* (Virus Research. 1995. **36**. 97-106) (GenBank AN # U10433).

Le plasmide pCMV-gD (Ambriovic A. *et al.* Virology. 1997. **238**. 327-335) a été digéré par SnaBI et Clal pour isoler le fragment SnaBI-Clal de 1,8 kpb (contenant la partie 3' du promoteur CMV et le gène PRV gD). Ce fragment a ensuite été traité avec l'ADN polymérase T4 pour le rendre bouts francs, puis il a été ligaturé avec le plasmide pE3dI622CMV-EGFP, préalablement digéré par SnaBI et Hpal, pour générer le plasmide pE3dI622CMV-gD (figure 2).

### 6.2. Construction du plasmide avec une délétion de 919 paires de bases (grande délétion) dans la région E3

Le plasmide pE3PAV3 (voir partie 6.1. *supra*) a été digéré par SgrAl et SacI pour isoler le fragment SgrAl-SacI de 644 pb. Ce fragment a été traité avec l'ADN polymérase T4 pour le rendre bouts francs, puis il a été ligaturé avec le plasmide pNEBPac- (6.1. *supra*)*,* préalablement digéré avec EcoRI, traité avec l'ADN polymérase T4, puis digéré avec Sacl, pour générer le plasmide pSgrAl-Sacl.

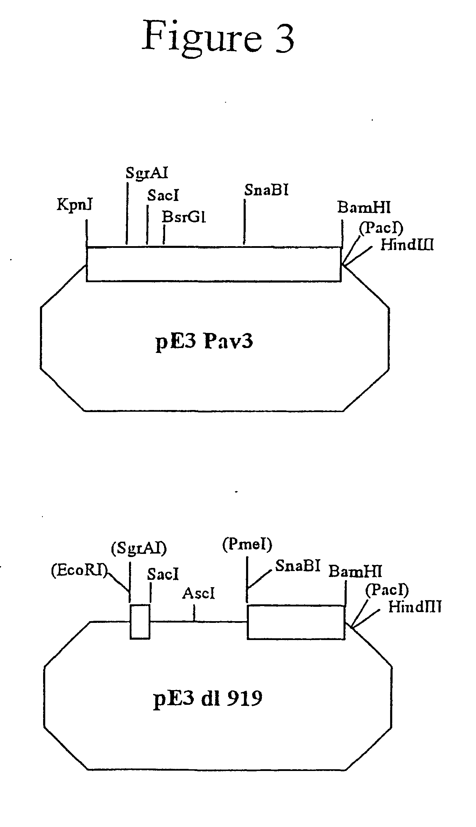

Le plasmide pE3PAV3 a été digéré par SnaBI et HindIII pour isoler le fragment SnaBI-HindIII de 2,4 kpb. Ce fragment a été ligaturé avec le plasmide pSgrAl-SacI, préalablement digéré avec PmeI et HindIII, pour générer le plasmide pE3dI919 (figure 3). La délétion créée entre les sites SacI et SnaBI de la région E3 du virus PAV-3 a une taille de 919 paires de bases. Cette délétion s'étend des nucléotides 706 à 1624 de la séquence publiée par P. Reddy *et al.* (Virus Research. 1995. 36. 97-106) (GenBank AN # U10433).

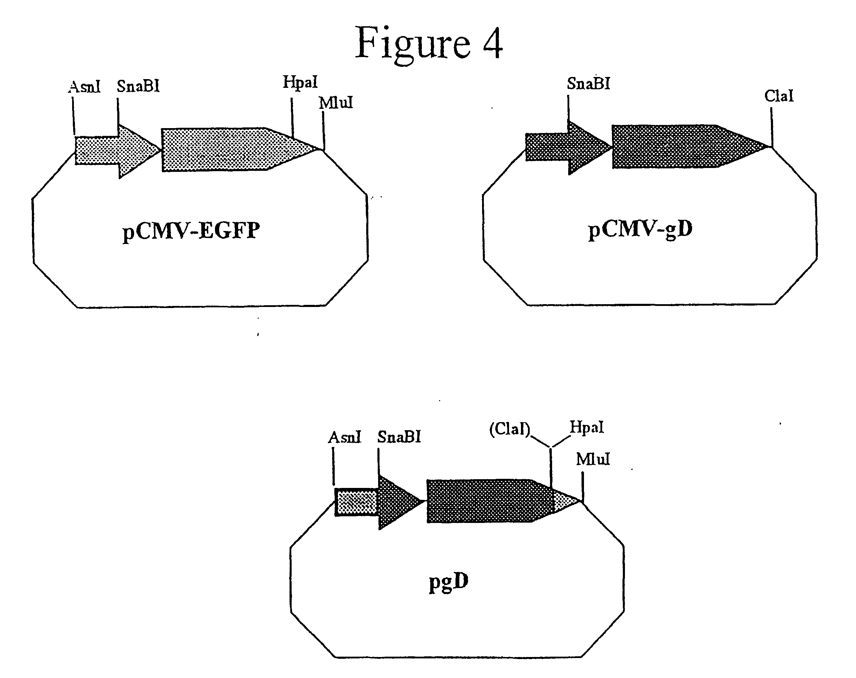

Le plasmide pCMV-gD (exemple 6.1) a été digéré par SnaBl et Clal pour isoler le fragment SnaBl-Clal de 1,8 kpb. Ce fragment a été alors traité avec le fragment Klenow de l'ADN polymérase pour le rendre bouts francs, puis il a été ligaturé avec le plasmide pCMV-EGFP (exemple 6.1), préalablement digéré avec SnaBl et Hpal puis déphosphorylé, pour générer le plasmide pgD (figure 4).

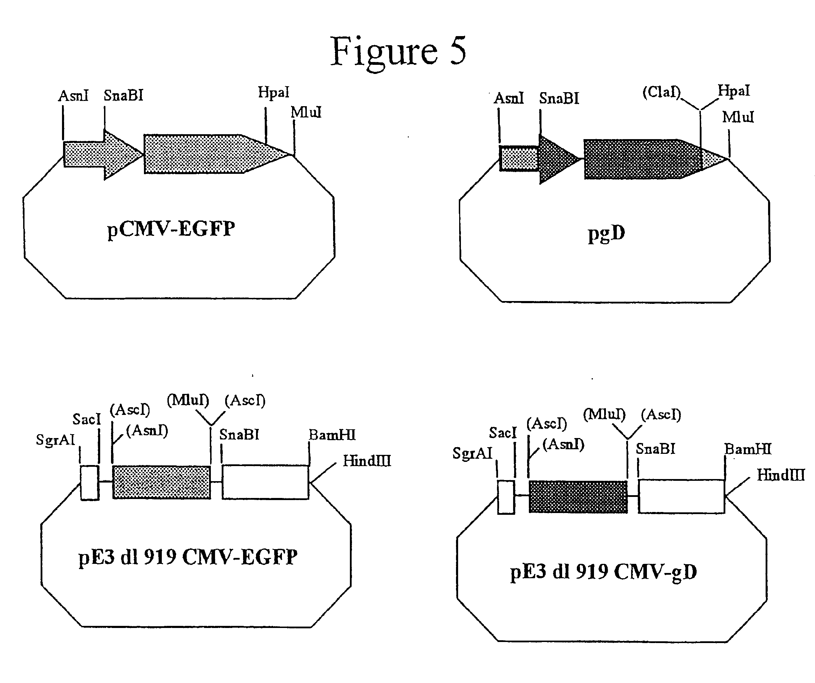

Le plasmide pCMV-EGFP (exemple 6.1) a été digéré par Asnl et Mlul pour isoler le fragment Asnl-Mlul de 1,8 kpb (cassette CMV-EGFP). Ce fragment a été traité avec le fragment Klenow de l'ADN polymérase pour le rendre bouts francs, puis il a été ligaturé avec le plasmide pE3dI919, préalablement digéré avec Ascl, traité avec le fragment Klenow de l'ADN polymérase pour le rendre bouts francs, puis déphosphorylé, pour générer le plasmide pE3dI919CMV-EGFP (figure 5).

Le plasmide pgD (*supra*) a été digéré par Asnl et Mlul pour isoler le fragment Asnl-Mlul de 2,3 kpb. Ce fragment a été traité avec l'ADN polymérase T4 pour le rendre bouts francs, puis il a été ligaturé avec le plasmide pE3dI919, préalablement digéré avec Ascl et traité avec le fragment Klenow de l'ADN polymérase pour le rendre bouts francs, puis déphosphorylé, pour générer le plasmide pE3dI919CMV-gD (figure 5).

### Exemple 7 : Construction des génomes recombinés pPAV3dI622CMV-EGFP et pPAV3dI622CMV-gD

Le plasmide pE3dI622CMV-EGFP (exemple 6.1) a été digéré par Kpnl et BamHI pour isoler le fragment KpnI-BamHI de 5,5 kpb. Ce fragment a été co-transformé dans des bactéries compétentes BJ5183 avec le plasmide pPAV3 (exemple 5) linéarisé par digestion avec l'enzyme SnaBI. Cette co-transformation a conduit à la génération, par recombinaison homologue dans *Escherichia coli,* du plasmide pPAV3dl622CMV-EGFP.

Le plasmide pE3CMVgD (exemple 6.1) a été digéré par EcoRI et PmeI pour isoler le fragment EcoRI-PmeI de 6,0 kpb. Ce fragment a été co-transformé dans des bactéries compétentes BJ5183 avec le plasmide pPAV3 (exemple 5) linéarisé par digestion avec l'enzyme SnaBI. Cette co-transformation a conduit à la génération, par recombinaison homologue dans *Escherichia coli,* du plasmide pPAV3d1622CMVgD.

### Exemple 8 : Construction des génomes recombinés pPAV3dI919CMV-EGFP et pPAV3dI919CMV-gD

Le plasmide pE3dI919CMV-EGFP (exemple 6.2) a été linéarisé par HindIII, puis co-transformé dans des bactéries compétentes BJ5183 avec le plasmide pPAV3 (exemple 5) linéarisé par digestion avec l'enzyme SnaBl. Cette co-transformation a conduit à la génération, par recombinaison homologue dans *Escherichia coli,* du plasmide pPAV3d)919CMV-EGFP.

Le plasmide pE3dI919CMV-gD (exemple 6.2) a été linéarisé par HindIII, puis co-transformé dans des bactéries compétentes BJ5183 avec le plasmide pPAV3 (exemple 5) linéarisé par digestion avec l'enzyme SnaBI. Cette co-transformation a conduit à la génération, par recombinaison homologue dans *Escherichia coli,* du plasmide pPAV3dl919CMV-gD.

### Exemple 9 : Construction dés génomes recombinés pPAV3dl622EGFP, pPAV3dl622gD, pPAV3dl919EGFP et pPAV3dl919gD (insertion des séquences codantes sans les séquences du promoteur CMV)

### 9.1. Construction des plasmides pE3dl622EGFP et pE3dl919EGFP

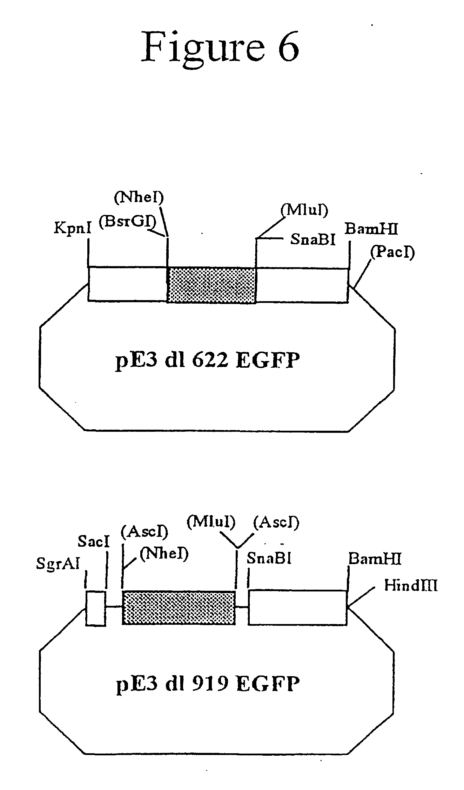

Le plasmide pCMV-EGFP (exemple 6.1) a été digéré par Nhel et Mlul pour isoler le fragment Nhel-Mlul de 1,0 kpb. Ce fragment a été traité avec le fragment Klenow de l'ADN polymérase pour le rendre bouts francs, puis ligaturé avec le plasmide pE3PAV3 (exemple 6.1), préalablement digéré par BsrGl, traité avec le fragment Klenow de l'ADN polymérase, et enfin digéré par SnaBl, pour générer le plasmide pE3dl622EGFP (figure 6).

Le plasmide pCMV-EGFP a été digéré par Nhel et Mlul pour isoler le fragment Nhel-Mlul de 1,0 kpb. Ce fragment a été traité avec le fragment Klenow de l'ADN polymérase pour le rendre bouts francs, puis ligaturé avec le plasmide pE3d1919, préalablement digéré par Ascl, traité avec le fragment Klenow de l'ADN polymérase, puis déphosphorylé, pour générer le plasmide pE3d1919EGFP (figure 6).

### 9.2. Construction des plasmides pE3dI622gD et pE3dI1919gD

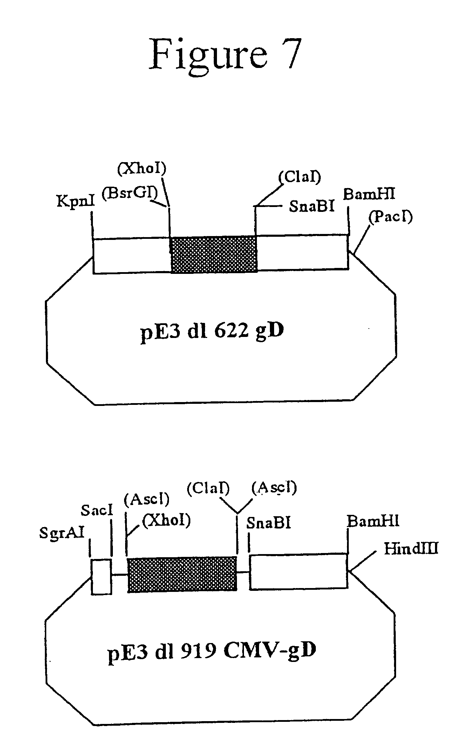

Le plasmide pgD (exemple 6.2) a été digéré par Xhol et Clal pour isoler le fragment Xhol-Clal de 1,5 kpb. Ce fragment a été traité avec le fragment Klenow de l'ADN polymérase pour le rendre bouts francs, puis il a été ligaturé avec le plasmide pE3PAV3 (exemple 6.1), préalablement digéré par BsrGl, traité avec le fragment Klenow de l'ADN polymérase, puis digéré par SnaBl, pour générer le plasmide pE3dI622gD (figure 7).

Le plasmide pgD (exemple 6.2) a été digéré par Xhol et Clal pour isoler le fragment Xhol-Clal de 1,5 kpb. Ce fragment a été traité avec le fragment Klenow de l'ADN polymérase pour le rendre bouts francs, puis il a été ligaturé avec le plasmide pE3dI919 (exemple 6.2), préalablement digéré par Ascl, traité avec le fragment Klenow de l'ADN polymérase, et enfin déphosphorylé, pour générer le plasmide pE3dl919gD (figure 7).

### 9.3. Construction des plasmides pPAV3dl622EGFP, pPAV3dl622gD, pPAV3dl919EGFP et pPAV3dl919gD

Le plasmide pE3dl622EGFP (exemple 9.1) a été digéré par Kpnl et BamHI, puis co-transformé dans des bactéries compétentes BJ5183 avec le plasmide pPAV3 (exemple 5), préalablement linéarisé par digestion avec SnaBI. Cette co-transformation a permis de générer, par recombinaison homologue dans *Escherichia coli* le plasmide pPAV3dI622EGFP.

Le plasmide pE3dI622gD (exemple 9.2) a été digéré par EcoRI et PmeI, puis co-transformé dans des bactéries compétentes BJ5183 avec le plasmide pPAV3 (exemple 5), préalablement linéarisé par digestion avec SnaBI. Cette co-transformation a permis de générer, par recombinaison homologue dans *Escherichia coli* le plasmide pPAV3d1622gD.

Le plasmide pE3dl919EGFP (exemple 9.1) a été linéarisé par digestion avec Hindlll, puis co-transformé dans des bactéries compétentes BJ5183 avec le plasmide pPAV3 (exemple 5), préalablement linéarisé par digestion avec SnaBI. Cette co-transformation a permis de générer, par recombinaison homologue dans *Escherichia coli* le plasmide pPAV3dl919EGFP.

Le plasmide pE3dl919gD (exemple 9.2) a été linéarisé par digestion avec Hindlll, puis co-transformé dans des bactéries compétentes BJ5183 avec le plasmide pPAV3 (exemple 5), préalablement linéarisé par digestion avec SnaBI. Cette co-transformation a permis de générer, par recombinaison homologue dans *Escherichia coli le* plasmide pPAV3dI919gD.

### Exemple 10 : Obtention des virus PAV3 recombinés par transfection avec les génomes PAV3 recombinés clonés dans Escherichia coli

Les cellules PK-15 ou ST (voir exemple 1) sont transfectées en plaque 6 puits à une densité de confluence de 60-80 % avec 2 µg d'ADN génomique PAV-3 recombiné, préalablement digéré par Pacl, dilué en présence de 4 à 12 µl de LipofectAMINE (Gibco-BRL Cat # 18324-012) selon la lignée cellulaire.

Après transfection selon les conditions recommandées par le fournisseur, les cellules sont laissées en culture pendant quelques jours jusqu'à l'apparition d'un ECP viral. Un nouveau passage en culture de cellules est alors réalisé pour amplifier le virus recombiné obtenu.
La transfection réalisée avec le plasmide pPAV3dI622CMV-EGFP (exemple 7) a généré le virus recombiné **vPAV3-1**.
La transfection réalisée avec le plasmide pPAV3dI622CMV-gD (exemple 7) a généré le virus recombiné **vPAV3-2**.
La transfection réalisée avec le plasmide pPAV3dI919CMV-EGFP (exemple 8) a généré le virus recombiné **vPAV3-3**.
La transfection réalisée avec le plasmide pPAV3dI919CMV-gD (exemple 8) a généré le virus recombiné **vPAV3-4**.
La transfection réalisée avec le plasmide pPAV3dI622EGFP (exemple 9.3) a généré le virus recombiné **vPAV3-5.**
La transfection réalisée avec le plasmide pPAV3dI622gD (exemple 9.3) a généré le virus recombiné **vPAV3-6**.
La transfection réalisée avec le plasmide pPAV3dl919EGFP (exemple 9.3) a généré le virus recombiné **vPAV3-7**.
La transfection réalisée avec le plasmide pPAV3dl919gD (exemple 9.3) a généré le virus recombiné **vPAV3-8**.

### Exemple 11 : Construction du plasmide pITRsPAV5

### 11.1. Clonage du fragment génomique terminal droit

L'ADN génomique du virus PAV-5, préparé selon l'exemple 3, a été digéré par EcoRI et le fragment terminal droit EcoRI I de 0,9 kpb a été isolé après électrophorèse en gel d'agarose. Ce fragment a été ligaturé avec le plasmide pBS-KS, préalablement digéré par EcoRI et déphosphorylé, pour générer le plasmide "pKS-Droit ITR5".

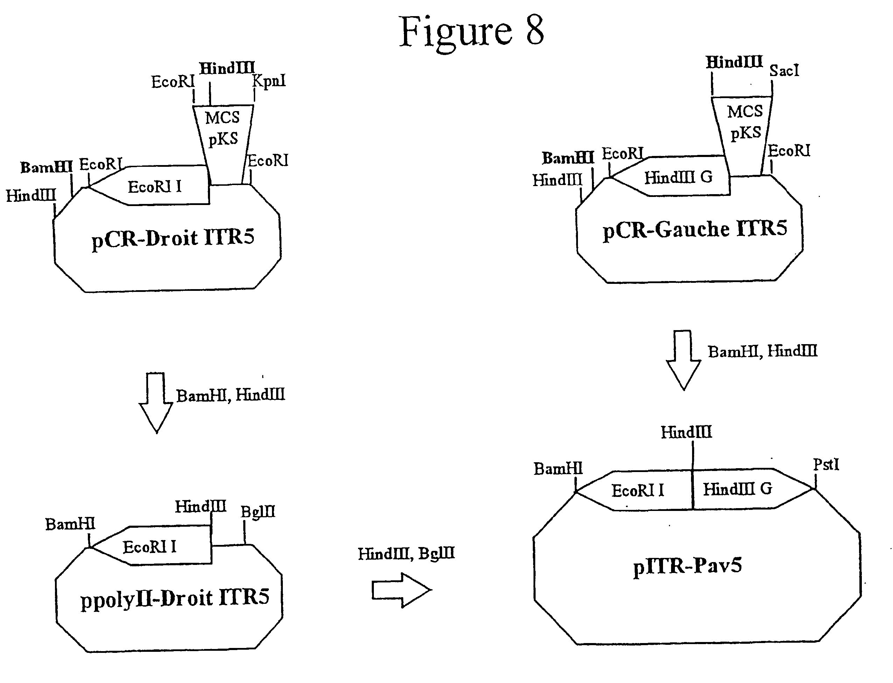

Une réaction ACP a alors été réalisée avec la matrice du plasmide pKS-Droit ITR5 et avec les oligonucléotides suivants :
ITRPAV5 (SEQ ID N°4) (50 mer) :
et T3 (SEQ ID N°2) (20 mer) :
pour produire un fragment de 1,0 kpb. Ce fragment a été ligaturé avec le plasmide pCRII (InVitrogen Original TA Cloning Kit Cat # K2000-01) pour donner le plasmide "pCR-Droit ITR5" (figure 8).

### 11.2. Clonage du fragment génomique terminal gauche

L'ADN génomique du virus PAV-5, préparé selon l'exemple 3, a été digéré par Hindlll et le fragment terminal gauche HindIII de 1,2 kpb a été isolé après électrophorèse en gel d'agarose. Ce fragment a été ligaturé avec le plasmide pBS-KS, préalablement digéré par HindIII et déphosphorylé, pour générer le plasmide "pKS-Gauche ITR5".

Une réaction ACP a alors été réalisée avec la matrice du plasmide pKS-Gauche ITR5 et avec les oligonucléotides suivants :
ITRPAV5 (SEQ ID N°4):
et T7 (SEQ ID N°3) (20 mer) :
pour produire un fragment de 1,3 kpb. Ce fragment a été ligaturé avec le plasmide pCRII pour donner le plasmide "pCR-Gauche ITR5" (figure 8).

### 11.3. Construction du plasmide pPolyli-Droit ITR5

Le plasmide pCR-Droit ITR5 (exemple 11.1) a été digéré par BamHI et HindIII pour isoler le fragment BamHI-HindIII de 1,0 kpb. Ce fragment a été ensuite ligaturé avec le plasmide pPolyll (voir exemple 4), préalablement digéré par BamHI et HindIII, pour générer le plasmide "pPolyll-Droit ITR5" (figure 8).

### 11.4. Construction du plasmide pITRsPAV5

Le plasmide pCR-Gauche ITR5 (exemple 11.2) a été digéré par BamHI et HindIII pour isoler le fragment BamHI-HindIII de 1,3 kpb. Ce fragment a été ensuite ligaturé avec le plasmide pPolyli-Droit ITR5, préalablement digéré par BgIII et HindIII, pour générer le plasmide "pITRs PAV5" (figure 8).

### Exemple 12 : Construction du plasmide pPAV5

Le plasmide piTRsPAV5 a été linéarisé par digestion avec HindIII. Le fragment obtenu a alors été co-transformé dans des bactéries compétentes BJ5183 avec l'ADN génomique du virus PAV-5 préparé selon l'exemple 3. Cette co-transformation a permis de générer par recombinaison homologue un plasmide contenant la totalité du génome du virus PAV-5. Ce plasmide a été désigné " pPAV5".

### Exemple 13 : Construction des plasmides Navette PAV-5

### 13.1. Construction du plasmide Navette E3 PAV-5

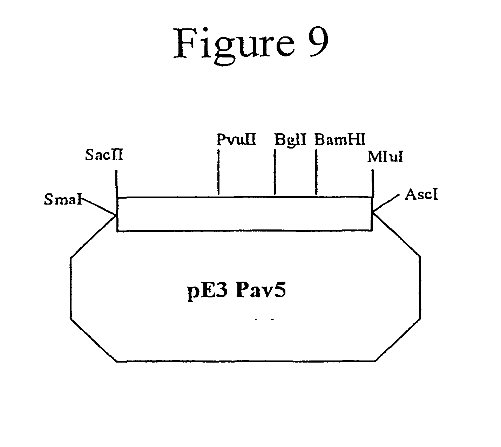

Le plasmide pPAV5 (exemple 12) a été digéré par SacI et MluI pour isoler le fragment SacI-MluI de 4372 pb contenant la région E3. Ce fragment a été ligaturé avec le plasmide pNEB193 (exemple 6.1), préalablement digéré par SmaI et AscI, pour donner le plasmide "pE3PAV5" (figure 9).

Le fragment SacI-MluI cloné dans le plasmide pE3P.AV5 a été entièrement séquencé et analysé. L'analyse de cette séquence et des séquences adjacentes présentes sur le plasmide pPAV5 a confirmé que le fragment cloné représentait bien la région E3 du virus PAV-5. La séquence de cette région (5614 paires de bases) (SEQ ID NO 5) est présentée sur la figure 13.

### 13.2. Construction des plasmides donneurs CMV-EGFP et CMV-PRV gD avec une délétion de 1472 paires de bases dans la région E3.

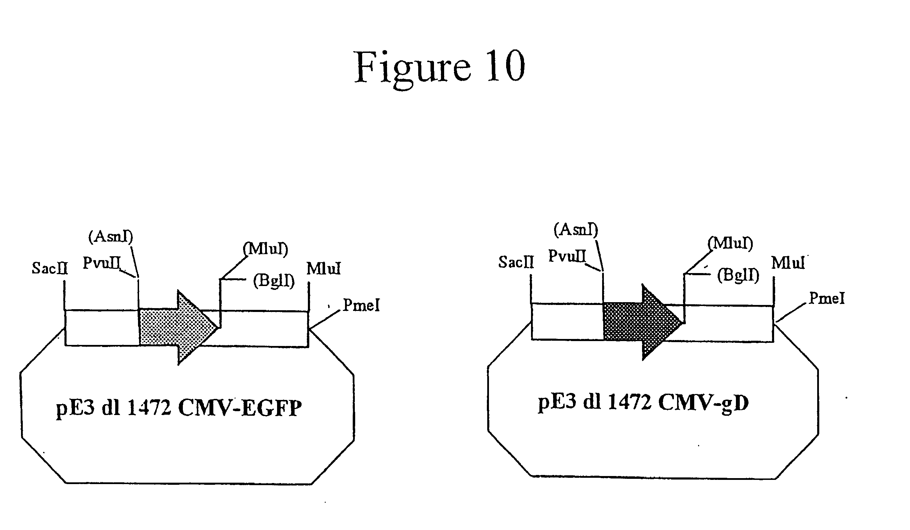

Le plasmide pCMV-EGFP (exemple 6.1) a été digéré par les enzymes Asnl et Mlul, puis traité avec l'ADN polymérase T4 pour isoler le fragment Asnl (bout franc) - Mlul (bout franc) de 1,6 kpb. Ce fragment a été ligaturé avec le plasmide pE3PAV5 (exemple 13.1), préalablement digéré par PvuII et BgII, et traité avec l'ADN polymérase T4, pour générer le plasmide pE3dl1472CMV-EGFP (figure 10). La délétion introduite entre les sites PvuII et BgII a une taille de 1472 pb. Cette délétion est comprise entre les nucléotides 2389 et 3861 de la séquence présentée sur la figure 13 (SEQ ID NO 5).

Le plasmide pgD (exemple 6.1) a été digéré par les enzymes Asnl et MluI pour isoler le fragment Asnl-Mlul de 2,3 kpb. Ce fragment a ensuite été traité avec l'ADN polymérase T4 pour le rendre bouts francs, puis il a été ligaturé avec le plasmide pE3PAV5, préalablement digéré par Pvull et BgII et traité avec l'ADN polymérase T4, pour générer le plasmide pE3dI1472CMVgD (figure 10).

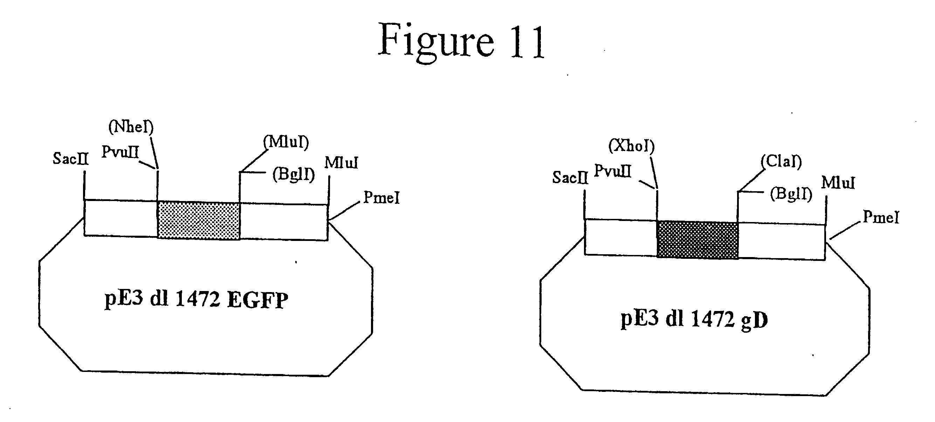

Le plasmide pCMV-EGFP a été digéré par Nhel et MluI pour isoler le fragment NheI-MluI de 1,0 kpb. Ce fragment a été traité avec le fragment Klenow de l'ADN polymérase pour le rendre bouts francs, puis ligaturé avec le plasmide pE3PAV5, préalablement digéré par Pvull et Bgll, traité avec l'ADN polymérase T4, pour générer le plasmide pE3dI1472EGFP (figure 11).

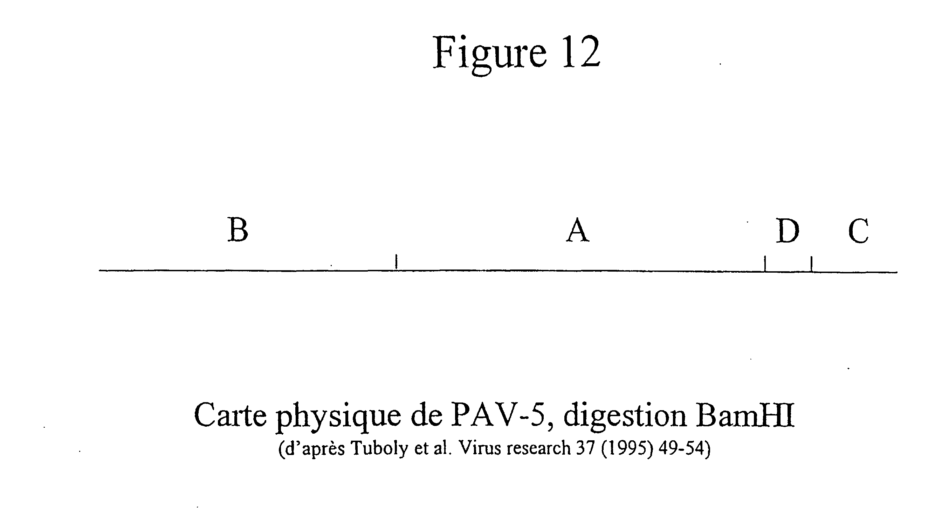

Le plasmide pCMVgD (exemple 6.1) a été digéré par Xhol et Clal pour isoler le fragment Xhol-Clal de 1,5 kpb. Ce fragment a été traité avec le fragment Klenow de l'ADN polymérase pour le rendre bouts francs, puis il a été ligaturé avec le plasmide pE3PAV5, préalablement digéré par PvuII et BgII, traité avec l'ADN polymérase T4, pour générer le plasmide pE3dI1472gD (figure 12).

### Exemple 14 : Construction des génomes recombinés pPAV5dI1472CMV-EGFP et pPAV5dI1472CMV-gD

Le plasmide pE3dI1472CMV-EGFP (exemple 13.2) a été linéarisé par Pmel, puis co-transformé dans des bactéries compétentes BJ5183 avec le plasmide pPAV5 (exemple 12) linéarisé par digestion partielle avec l'enzyme BamHI (site de jonction des sous-fragments de restriction BamHI A-BamHI D, figure 12). Cette co-transformation a conduit à la génération, par recombinaison homologue dans *Escherichia coli,* du plasmide pPAV5dI1472CMV-EGFP.

Le plasmide pE3dI1472CMVgD (exemple 13.2) a été linéarisé par Pmel, puis co-transformé dans des bactéries compétentes BJ5183 avec le plasmide pPAV5 (exemple 12) linéarisé par digestion partielle avec l'enzyme BamHI (site de jonction des sous-fragments de restriction BamHI A-BamHI D, figure 12). Cette co-transformation a conduit à la génération, par recombinaison homologue dans *Escherichia coli,* du plasmide pPAV5dI1472CMV-gD.

### Exemple 15 : Construction des génomes recombinés pPAV5dI1472EGFP et pPAV5dI1472 gD (insertion des séquences codantes sans les séquences du promoteur CMV)

Le plasmide pE3dI1472EGFP (exemple 13.2) a été linéarisé par Pmel, puis co-transformé dans des bactéries compétentes BJ5183 avec le plasmide pPAV5 (exemple 12) linéarisé par digestion partielle avec l'enzyme BamHI (site de jonction des sous-fragments de restriction BamHI A-BamHI D, figure 12). Cette co-transformation a conduit à la génération, par recombinaison homologue dans *Escherichia coli,* du plasmide pPAV5dI1472EGFP.

Le plasmide pE3dI1472gD (exemple 13.2) a été linéarisé par Pmel, puis co-transformé dans des bactéries compétentes BJ5183 avec le plasmide pPAV5 (exemple 12) linéarisé par digestion partielle avec l'enzyme BamHI (site de jonction des sous-fragments de restriction BamHI A-BamHI D, figure 12). Cette co-transformation a conduit à la génération, par recombinaison homologue dans *Eschérichia coli,* du plasmide pPAV5dIl472gD.

### Exemple 16 : Obtention des virus PAV-5 recombinés par transfection avec les génomes PAV-5 recombinés clonés dans Escherichia coli

Les cellules PK-15 ou ST (voir exemple 1) sont transfectées en plaque 6 puits à une densité de confluence de 60-80 % avec 2 µg d'ADN génomique PAV-5 recombiné, préalablement digéré par Pacl, dilué en présence de 4 à 12 µl de LipofectAMINE (Gibco-BRL Cat # 18324-012) selon les lignées cellulaires.

Après transfection selon les conditions recommandées par le fournisseur, les cellules sont laissées en culture quelques jours jusqu'à l'apparition d'un ECP viral. Un passage est alors réalisé pour amplifier le virus recombiné obtenu.
La transfection réalisée avec le plasmide pPAV5dI1472CMV-EGFP (exemple 14) a généré le virus recombiné **vPAV5-1.**
La transfection réalisée avec le plasmide pPAV5dI1472CMV-gD (exemple 14) a généré le virus recombiné **vPAV5-2**.
La transfection réalisée avec le plasmide pPAV5dl1472EGFP (exemple 15) a généré le virus recombiné **vPAV5-3**.
La transfection réalisée avec le plasmide pPAV5dl1472gD (exemple 15) a généré le virus recombiné **vPAV5-4**.

### Exemple 19 : Fabrication des vaccins selon l'invention

Les virus recombinés obtenus selon l'invention sont cultivés et amplifiés en culture de cellules PK-15. Les surnageants sont récoltés après observation d'un effet cytopathogène complet. Après clarification par centrifugation à basse vitesse pour éliminer les débris cellulaires, puis centrifugation pour concentrer les virions et lavage du culot viral, le culot est repris par une solution de milieu de culture. Le titre viral de ces suspensions est mesuré. Le titre viral est alors éventuellement ajusté par dilution de manière à obtenir un titre viral final compris entre 10⁴ doses infectantes 50 % en culture de cellules (DICC50) et 10⁷ DICC50/ml.

Les suspensions virales peuvent être soit congelées, soit de préférence lyophilisées en présence d'un substrat de lyophilisation. **Exemple 20 : Vaccination des porcs**

Après décongélation ou après reprise des doses lyophilisées en eau pour préparations injectables, et addition éventuelle d'un adjuvant, les porcs sont vaccinés avec des doses de 10⁴ à 10⁷ DICC50 de chaque virus recombiné. Les vaccins comprennent un ou plusieurs virus PAV recombinés exprimant chacun des immunogènes différents.

Les vaccins sont administrés soit par injection à l'aiguille (voie intramusculaire) sous un volume de 2 ml, soit par voie muqueuse (voie intranasale ou instillation oculaire) (volumes adaptés à chaque voie).

Les injections par voie intradermique sont également réalisables au moyen d'un injecteur sans aiguille tel que l'appareil PIGJET (Société Endoscoptic, Laon, France).

### Exemple 21 : Protocole de vaccination/épreuve maladie d'Aujeszky

Un protocole de vaccination/épreuve a été mis en oeuvre pour évaluer l'efficacité d'un vaccin constitué par une suspension du virus recombiné vPAV3-2 non adjuvé (exemple 10 de la présente invention).

Des groupes de 6 porcelets de 8 à 10 semaines d'âge, nés de truies ayant été vaccinées contre le virus de la maladie d'Aujeszky (PRV) en début de gestation, ont été constitués. Tous les porcelets possédaient bien des anticorps maternels anti-PRV au jour J0 de la vaccination. Les porcelets des groupes 1 et 2 ont été vaccinés à J0 par voie intranasale avec 1 millilitre (ml) d'une suspension du virus recombiné vPAV3-2 (PAV-3/PRV gD) titrant 10⁷ DICC50/ml (0,5 ml par narine). Le groupe 2 a été vacciné de la même façon, mais a également reçu une deuxième administration à J21. Le groupe 3 a reçu à J0 par voie intramusculaire 1 ml d'une suspension virale de PAV-3/gD titrant 10⁷ DICC50/ml. Le groupe 4 a été vacciné de la même façon que le groupe 3, mais a reçu une seconde injection de suspension virale à J21. Le groupe 5 n'a pas été vacciné et a servi de groupe témoin négatif pour l'épreuve.

Tous les groupes ont été éprouvés à J35 par administration de 2 ml (1 ml par narine) d'une suspension de la souche PRV NIA3 titrant au moins 10^{7,3} pfu/ml.

Après épreuve, les porcs ont été suivis sur les critères d'évolution pondérale (delta G7) (Stellmann C*. et al.* J. Biol. Standard. 1989. **17**. 1-11) (J0 à J7 après épreuve), de niveau d'excrétion virale au niveau des muqueuses nasales, et sur les titres anticorps ELISA et séroneutralisants anti-PRV.

Il doit être bien compris que l'invention définie par les revendications annexées n'est pas limitée aux modes de réalisation particuliers indiqués dans la description ci-dessus.

## Revendications

1. Un adénovirus porcin de sérotype 3 recombiné (PAV-3), qui contient au moins un acide nucléique hétérologue codant pour au moins un immunogène, l'acide nucléique hétérologue étant inséré dans la région E3 du génome de PAV-3, située entre le gène codant pour la protéine pVIII et celui codant pour la fibre, l'adénovirus recombiné étant capable de se répliquer *in vivo* et d'exprimer l'immunogène, l'immunogéne étant choisi dans le groupe consistant en : immunogène du circovirus porcin de type 2, gB du virus de la maladie d'Aujeszky, gC du virus de la maladie d'Aujeszky, immunogène du virus de la grippe porcine, VP2 du virus de la parvovirose, ORF3 du virus de la maladie mystérieuse du porc, ORF4 du virus de la maladie mystérieuse du porc, ORF6 du virus de la maladie mystérieuse du porc, ORF7 du virus de la maladie mystérieuse du porc, E0 du virus de la peste porcine classique, E1 du virus de la peste porcine classique et immunogène d*'Actinobacillus pleuropneumoniae.*

2. L'adénovirus selon la revendication 1, dans lequel l'acide nucléique hétérologue est inséré dans la région E3 du génome de PAV-3, dans une zone d'insertion qui, dans la souche 6618, a la séquence représentée par SEQ ID NO :6.

3. L'adénovirus selon l'une quelconque des revendications 1 ou 2, dans lequel la zone d'insertion comporte une délétion.

4. L'adénovirus selon l'une quelconque des revendications 1 à 3, dans lequel l'acide nucléique hétérologue code pour au moins un immunogène du circovirus porcin de type 2.

5. L'adénovirus selon la revendication 4, dans lequel l'acide nucléique hétérologue code pour ORF1, ORF2 ou ORF1 et ORF2 du circovirus porcin de type 2.

6. L'adénovirus selon l'une quelconque des revendications 1 à 5, dans lequel l'acide nucléique hétérologue code pour HA, NA et/ou NP du virus de la grippe porcine.

7. Adenovirus porcin selon l'une des revendications 1 à 6, comprenant une séquence nucléotidique hétérologue codant pour une cytokine porcine.

8. Adénovirus selon la revendication 7, dans lequel la cytokine porcine est choisie dans le groupe consistant en GM-CSF, IL-4, IL-12 et IL-18.

9. Vaccin recombiné porcin comprenant un adénovirus porcin PAV-3 recombiné selon l'une quelconque des revendications 1 à 8, et un véhicule ou excipient acceptable sur le plan vétérinaire.

10. Vaccin recombiné porcin selon la revendication 9, comprenant plusieurs adénovirus porcins PAV-3 recombiné selon l'une quelconque des revendications 1 à 8, lesquels comprennent des séquences nucléotidiques hétérologues différentes.

11. Vaccin recombiné porcin selon la revendication 9 ou 10, comprenant un adjuvant.

12. Vaccin recombiné porcin selon la revendication 11, dans lequel l'adjuvant est choisi parmi les polymères de l'acide acrylique ou méthacrylique et les copolymères d'anhydride maléique et de dérivé alcényle.

13. Vaccin recombiné porcin selon la revendication 12, dans lequel l'adjuvant est un carbomère.

## Patentansprüche

1. Rekombinantes Schweine-Adenovirus vom Serotyp 3 (PAV-3), das mindestens eine heterologe Nucleinsäure enthält, die mindestens ein Immunogen codiert, wobei die heterologe Nucleinsäure in die E3-Region des PAV-3-Genoms inseriert wurde, positioniert zwischen dem Gen, welches das Protein VIII codiert, und dem Gen, welches die Faser codiert, wobei das rekombinante Adenovirus in der Lage ist, *in vivo* zu replizieren und das Immunogen zu exprimieren,
wobei das Immunogen ausgewählt ist aus der Gruppe bestehend aus: dem Immunogen des porcinen Circovirus Typ 2, dem gB des Virus der Aujeszky'schen Krankheit, dem gC des Virus der Aujeszky'schen Krankheit, dem Immunogen des Schweinegrippevirus, dem VP2 des Parvovirose-Virus, dem ORF3 des Virus der mysteriösen Schweinekrankheit (seuchenhafter Spätabort der Schweine, MSD-Krankheit), dem ORF4 des Virus der MSD-Krankheit, dem ORF6 des Virus der MSD-Krankheit, dem ORF7 des Virus der MSD-Krankheit, dem E0 des Virus der klassischen Schweinepest, dem E1 des Virus der klassischen Schweinepest und dem Immunogen von *Actinobacillus pleuropneumoniae.*

2. Adenovirus nach Anspruch 1, bei dem die heterologe Nucleinsäure in die E3-Region des PAV-3-Genoms in einem Insertionsbereich inseriert wurde, der im Stamm 6618 durch die Sequenz SEQ ID No: 6 dargestellt ist.

3. Adenovirus nach einem der Ansprüche 1 oder 2, bei dem der Insertionsbereich eine Deletion aufweist.

4. Adenovirus nach eine der Ansprüche 1 bis 3, bei dem die heterologe Nucleinsäure mindestens ein Immunogen des porcinen Circovirus Typ 2 codiert.

5. Adenovirus nach Anspruch 4, bei dem die heterologe Nucleinsäure ORF1, ORF2 oder ORF1 und ORF2 des porcinen Circovirus Typ 2 codiert.

6. Adenovirus nach einem der Ansprüche 1 bis 5, bei dem die heterologe Nucleinsäure HA, NA und/oder NP des Schweinegrippevirus codiert.

7. Schweine-Adenovirus nach einem der Ansprüche 1 bis 6, das eine heterologe Nucleotidsequenz umfasst, die ein Cytokin vom Schwein codiert.

8. Adenovirus nach Anspruch 7, bei dem das Cytokin vom Schwein ausgewählt ist aus der Gruppe bestehend aus GM-CSF, II-4, IL-12 und IL-18.

9. Rekombinanter Schweineimpfstoff, der ein rekombinantes Schweine-Adenovirus PAV-3 nach einem der Ansprüche 1 bis 8 und einen Träger oder Exzipienten umfasst, der in der Veterinärmedizin verträglich ist.

10. Rekombinanter Schweineimpfstoff nach Anspruch 9, der mehrere rekombinante Schweine-Adenoviren PAV-3 nach einem der Ansprüche 1 bis 8 umfasst, die unterschiedliche heterologe Nucleotidsequenzen umfassen.

11. Rekombinanter Schweineimpfstoff nach Anspruch 9 oder 10, der ein Adjuvans umfasst.

12. Rekombinanter Schweineimpfstoff nach Anspruch 11, wobei das Adjuvans ausgewählt ist aus Acrylsäure- oder Methacrylsäurepolymeren und den Copolymeren von Maleinsäureanhydrid und den Alkenylderivaten.

13. Rekombinanter Schweineimpfstoff nach Anspruch 12, bei dem das Adjuvans ein Carbomer ist.

## Claims

1. A recombinant porcine adenovirus serotype 3 (PAV-3), which contains at least one heterologous nucleic acid coding for at least one immunogen, the heterologous nucleic acid being inserted into the E3 region of the PAV-3 genome, located between the gene coding for the pVIII protein and that coding for fibre, the recombinant adenovirus being capable of replicating *in vivo* and expressing the immunogen, the immunogen being selected from the group consisting of: porcine circovirus type 2 immunogen, pseudorabies virus gB, pseudorabies virus gC, swine influenza virus immunogen, parvovirus VP2, porcine reproductive and respiratory syndrome ORF3, porcine reproductive and respiratory syndrome ORF4, porcine reproductive and respiratory syndrome ORF6, porcine reproductive and respiratory syndrome ORF7, hog cholera virus E0, hog cholera virus E1 and *Actinobacillus pleuropneumoniae* immunogen.

2. Adenovirus according to claim 1, in which the heterologous nucleic acid is inserted into the E3 region of the PAV-3 genome, into an insertion zone which, in the 6618 strain, has the sequence represented by SEQ ID NO: 6.

3. Adenovirus according to claim 1 or claim 2, in which the insertion zone comprises a deletion.

4. Adenovirus according to any one of claims 1 to 3, in which the heterologous nucleic acid codes for at least one porcine circovirus type 2 immunogen.

5. Adenovirus according to claim 4, in which the heterologous nucleic acid codes for ORF1, ORF2 or ORF1 and ORF2 of porcine circovirus type 2.

6. Adenovirus according to any one of claims 1 to 5, in which the heterologous nucleic acid codes for HA, NA and/or NP of the porcine influenza virus.

7. Porcine adenovirus according to one of claims 1 to 6, comprising a heterologous nucleotide sequence coding for a porcine cytokine.

8. Adenovirus according to claim 7, in which the porcine cytokine is selected from the group consisting of GM-CSF, IL-4, IL-12 and IL-18.

9. A recombinant porcine vaccine comprising a recombinant porcine adenovirus PAV-3 according to any one of claims 1 to 8, and a vehicle or excipient which is acceptable from a veterinary viewpoint.

10. A recombinant porcine vaccine according to claim 9, comprising a plurality of recombinant porcine adenoviruses PAV-3 according to any one of claims 1 to 8, comprising different heterologous nucleotide sequences.

11. A recombinant porcine virus according to claim 9 or claim 10, comprising an adjuvant.

12. A recombinant porcine virus according to claim 11, in which the adjuvant is selected from acrylic acid or methacrylic acid polymers and from copolymers of maleic anhydride and alkenyl derivative.

13. A recombinant porcine virus according to claim 12, in which the adjuvant is a carbomer.


### SEQUENCE LISTING

<110> MERIAL Ecole Nationale Vétérinaire de Maisons ALFORT
<120> Recombined porcine adenovirus based viral vaccines and vec tors
<130> PAV EP
<140> EP 00/903750
   <141> 2000-02-08
<150> FR 99/01813
   <151> 1999-02-11
<160> 6
<170> PatentIn version 3.2
<210> 1
   <211> 50
   <212> DNA
   <213> Artificial Sequence
<220>
   <223> oligonucleotide
<400> 1
   ccttaattaa ggtagggata acagggtaat catcatcaat aatataccgc
        50
<210> 2
   <211> 20
   <212> DNA
   <213> Artificial Sequence
<220>
   <223> oligonucleotide
<400> 2
   attaaccctc actaaaggga
   20
<210> 3
   <211> 20
   <212> DNA
   <213> Artificial Sequence
<220>
<223> oligonucleotide
<400> 3
   taatacgact cactataggg
   20
<210> 4
   <211> 50
   <212> DNA
   <213> Artificial Sequence
<220>
   <223> oligonucleotide
<400> 4
   ccttaattaa ggtagggata acagggtaat catcatcaat aatatacgga
   50
<210> 5
   <211> 5614
   <212> DNA
   <213> Porcine adenovirus 5
<400> 5
   aagcttctct gccagattta acgagccaaa gcatgatgca gcaatttaga aactttaccc 60
   tcgaaaggtc tggcattctt ccggcctgct gcaacgcctt tcccacggac tttgtaccca 120
   tcacctataa agaatgccca ccccctctct ggggatactg ctacctcatg aggctggcca 180
   actttatcat gttccacacc gatgtggctt acaacatgga aggggagggc ttgttcgaat 240
   gctattgccg gtgcaacctt tgcacccctc accgatgctt ggccaccaac accgccctgt 300
   taaacgaggt tcaggccatc ggcactttcg aacttcaagg tccaccaaac gaggatgggt 360
   ccatgccaca cccactcaaa ctcacggccg gcgcctggac gagcgcctac cttaaaaagt 420
   ttgaggaaaa agactactgt caccacacca tccaatttta cgaagaccaa tcaaaagcac 480
   ccaaagcaga gttgaccgcc tgtgtcatca ctcaagccgc catcctcgcc caattacatg 540
   acattaaaaa agcgcgggaa aacttcctcc ttaaaaaggg gcacggggta tacctcgacc 600
   cacagaccgg agaggaactc aacacgtcca ccccctccgc cgccaacaat gccgaagcta 660
   aaaactcctc gaccgggttc cctaaatctg acaaacccca tcatgaagaa gagcagaaaa 720
   accaaacaga ttctgccgcc gcctcctcca actccagaag aagaggagat tatagacgag 780
   gaggccgagg aatgggacga agagagcatg gattctcagg aaggattgga aaccatcgaa 840
   gagctggagg aaggggaaat ccctcctacc cctccaacca ttcccaaaaa gcagcgtaga 900
   tgggatcaga aacccgaatt gatcaacgcg cagaccggag gtaaggccga gtcaagatat 960
   aagtcctggc gcagatacaa aaatattatt tataaaaccc tgatggccag cggctacgac 1020
   gtctctttcg cgcgccgcta tcttctgttc agacacgggg tcaatattcc gaaaaatgta 1080
   atccattact acaattccca ctgcagaaac caagaccccg aagaagtctg gaaggaaatc 1140
   catccagtct gccaatatat ccaaagaacc agcgacgacc agagcgctaa gagctaggat 1200
   ctttcccacg ttgtatgcca tcttccagca gagccgcgga gtagacagca acctcaaggt 1260
   taaaaacaga tcacttcgct ctctcaccaa aagctgcttg tatcacaacc aggaatctca 1320
   gcttcaaaga accttagagg acgctgaagc tctccttcac aagtactgct ccggtctgac 1380
   tgcctcctct tataatgagt aaagacatcc ccacccctta cgtgtggacc tatcagcctc 1440
   agctaggtca ggcggctggc gcgtcacaag actactccac ccgcatgaat tggctaagtg 1500
   ccggaccgtc aatgattgac caggtgaatc agataagggt ggaacgcaat aatattttat 1560
   tgcggcaggc ggcggccacg gagacgccca ggttagtgcg gaatccaccc aactggcccg 1620
   cacgctacct gtaccagccc atgggtgcgc cgcaaacagt ggaacttccg cgtaatgagt 1680
   tactcgaaac cgtcatgact aactctggaa tgcagttggc gggcggaggt cgcactacct 1740
   gcggtataaa aggagcacac ctgagcggct caggtattca gctcaacgga gagctaccga 1800
   gcgcctcttg gctgagacct gacggagtct tccagctagc tggtggcagc cgttcttctt 1860
   tcagcccagg cgtcagcact ctgctgagac tagaaccatc ttcctcccta ccgagatccg 1920
   gtgggattgg aagcacccaa ttcgttcaag agtttgttcc agccgtctac tttcagcctt 1980
   tctccggacc acctggaaca tatcctgacg aattcatcta caactacgac atagtctctg 2040
   actctgtcga cggttatgac tgatagagac gccagacttg ctgtcaccgc tctctcatcc 2100
   ctgcatcaag agacctgcaa gagaagccac tgcttcacca aggccgacct caacctagcc 2160
   tacttctgtg tgctgcccag cgaccttgaa gatgattgtg tgcctgactc cctccaagtc 2220
   ggacacgggc ttcggcttga gcttccgtac tgcttcaaaa gctttttcat ctttaaggga 2280
   ggaaaaaaat acctctgcag cgaacacctc caaaatggcc acctcaaagt ctcgtgcgac 2340
   tgcgcctccc ctggcgccca tctcgacatg tttgacacac tttgccagct gtataacaac 2400
   actgctcctc gtcctgattc tgtctcatct gactccgacg actgaagccg cccagccaga 2460
   agtgcgcatc aaggcggtca cttacgactg gctgtgtgtg cttgacatca actgcactca 2520
   ccagcagcct gtacacttaa aatggaaggg gtatgattac aagacaagcc atctcaccat 2580
   caaagcaaac tcagacatct cagacgagcc cgtcatgtgt accacagaca accatcccag
   2640
   agaaacggcc actgttgaca ttcgagacca ctgcaaattt ccaccgaccg ttagatacat
   2700
   gctgaagaaa gtcctaccca tctacttcac ctttctgtca ggtgtgatgc tatgtctcgt
   2760
   gtttcctgca gctagatacc catcacttct gtcggctctt ccagttgcca tggctagcac
   2820
   cacatctagc atcactactg atgtgagtac tctgtcaaac tactccagcg aacaatctac
   2880
   aaccgaacat ccaaccacag caaattcaca gccttacacg gccgtggaga agacactgct
   2940
   tgctttgatt ccgatcctca ttgcctgcat tgtaatggtg cttgttttca catttctgct
   3000
   aaagaagaaa cagaaggaag taaatgacgc tggagtctac cccatataca gaccaagaag
   3060
   gtcgtcatcc atgtcaaagc taacacttat tactacactg tgtttactgg caaccgcaaa
   3120
   ctgcgaaaaa cactacaaat cttatgttga aggagaaaac gcctatcttg aaaatctatt
   3180
   taacaactac tgtgagctaa agtggtattg gttttatacg agacatgagg gatatgtttt
   3240
   ggcaaaacgc gatggcgacc acactcgtac atttgattta aaaccaaaaa acatgtatgt
   3300
   agatgaagat acttttgacc tgattataag aaacattgaa gtaaaaaaca ctggaatgta
   3360
   tgaagtagaa tatcactgtg gcacatatac aaggtctgag tactatgtag tcacagttct
   3420
   tccaaacatc gcagaatcat ctctaaacgt tgaatctgtg tctatcaacg gcagcatttg
   3480
   tcaaatctct ttacattgtg gcatcgagaa caacttagtg gaaacaagaa ttatatatga
   3540
   ggacaatgtc attagtgaca acaatcttat gctcagcttg aacatctcta aagaacccag
   3600
   cacactcact tgctttacta acacttctaa acaatctgta aaactaagtt tgaatatatc
   3660
   tgattattgc aaacaaccta attcaaaaca tgaaaaaatc atcctaagtg aagattcttc
   3720
   agaaaatgaa aagaagcaaa taatcgtttt ttcggtcaca gcggcagttg cattcctaat
   3780
   gattattcta ctgataatat ctttcaaacc aagactttgc ttcggaactc cagtatatgc
   3840
   agtcgtattg gctgccacag gggcagaagc ctcaaattgt acttgtgctg atgttaacaa
   3900
   cgattggtca tatgtcctag ttatcattct ttcaactcta acttgtatca gtctaattgt
   3960
   tggaattcta aagattattt ttctttgcct cttccgctgc ccaggcaatc atgatgatga
   4020
   tgatgtctaa ttgaaatcac taataaagat ttctaaacta aaattggctt tttcgttttt
   4080
   agcatgaaac gcgcaaagag atcaatacct acagactttg atccggttta tccatacggg
   4140
   aacccaccac ttaacataat tcctccattt tacagtacag atggttttca agagttccca
   4200
   gttacaacac tatctcttaa agtagacgac cctgttacct tttccaacac tgggggcatc
   4260
   acactgaaat tgggtggtgg tgttagcatc aaccaaaacg gagaactaga atcatcgtcc
   4320
   gtaccaacta tactaaatcc accacttgac aattccaacg gatcccttag cttaaatata
   4380
   ggagagggtt tacaggaaag caatggagca cttgtactct acaaagaacc accattcatt
   4440
   ttctcaagca acgcgctagg tatagatttg ggtaatggca tgcagctatc ggcagataaa
   4500
   ctgtcactaa aattgggaaa cggcttatcc ttttcatcag atggctcctt acaagttcaa
   4560
   acttcaccgc ctctgataaa tgggtcaagt attggtctaa acatcgcaaa ccctttttca
   4620
   atagactcaa atcaggcatt gtctttgcaa acggaatcat atttttctac aaacaactca
   4680
   ctttctttaa atgttggaga tggattgcaa accacaaaca accagctatc gcttcaggtg 4740
   tcaccttact ttgggttttc atctgggtcc ctgtccttat ccattgctaa catgatgaac 4800
   ctcaggaaca acactctagg agtaaatgtt ggcaacggcc tgtttgttaa tccttcgaac 4860
   gcaatagctg tcaatgttag agcgccttta aactatagcg gtactacaaa atccgtaacc 4920
   gttgtagccg gaccaggcct caccatcagc ggggaaacac ttggctctga tataagagtg 4980
   aatgctggca atgggttgtt tgctgataca caaaatgtaa gggtaaaact aggtgccggg 5040
   cttatttttg actcaaatgg aaatattcaa gtaaatgtgg gttctggatt gcaaatacag 5100
   aacaatgctg tggtggtggc ctcatctacc aacacctcct catccattgc aagttactca 5160
   gcaagttcag catctactcc cacatcgaca tcgcccacta gcgggacata tctctacaac 5220
   tatcaaatag cctttagttg ggacattgta gaaaccgatc aaaactattt catctacacg 5280
   ctcagatgca ccgagattac gcctcaaaat aatcaggtcg aactcagctt cacaccgaca 5340
   gacccaaact ttataagttt ctttgactac ataaacacga tgcacaccgt tgcgcatcaa 5400
   atttctggtt ctactgtaac caatataccc atcaccgtga cgtacagtaa ttctagcaac 5460
   caactgagga ttatcttctc ctccacggtg gttaaaaacc taacaatgac accttgggtg 5520
   gcgagcgttg tcaggcataa agcaaccatt actagtggaa gtgcgtggat gggcacggct 5580
   tggtagccgg cttacctgta aagtttaaac gcgt 5614
<210> 6
   <211> 623
   <212> DNA
<213> Porcine adenovirus 3
<400> 6
   tgtacaaccg ctcgtagcgt cagcccctac acctcccctc gcgtccaatt tctgtccgac
        60
   atagaaccag actctgactc ttactcgggc tctggctctg gggacgatga agattatgaa
       120
   tatgagctgg ctaccaacac accgaacgaa gacattctag gcagcatagt catcaacaac
       180
   cagatcgggc ccaagaccct ggccctggga tacttttatg ccgccatgca gtttgtcttc
       240
   tttgccatca tcatcatcgt cctcatcctc tactaccgcc gctacgtgct ggccaccgcc
       300
   ctcatcgtgc agcgccagat gtggtcctcc gaggccgtcc tgcggaaaac cttctcggcc
       360
   accgttgtgg ttactccccc aaaacaagtc accccctgca actgctcctg ccgcttcgag
       420
   gagatggtgt tctactacac cacctccgtc ttcatgccct ggtgggcctc atcctcctgc
       480
   tcaccgccat ggtccgcctg gccaactgga tagtggatca gatgcccagc aggaaccgcg
       540
   ccccgccgct gccaccgccc ctcacctatg tgggaccctg cgccgaggac cacatctacg
       600
   atgagccaac cgtagggcaa tac
       623